# EUROPEAN PATENT SPECIFICATION

(11) **EP 2 894 148 B1**
(45) Date of publication and mention of the grant of the patent: **03.04.2019**
(21) Application number: 12884098.0
(22) Date of filing: 27.09.2012
(51) Int. Cl.: C07D 213/16, C07D 237/08, C07D 239/26, C07D 319/06, C07D 339/08, C09K 19/34, C07F 5/02, C07D 213/79, C07D 213/80, C07D 213/85, C07D 213/53, C07D 213/57, C09K 19/40, C09K 19/52, G02F 1/13

(54) **NOVEL SMECTIC PHASE A LIQUID CRYSTAL MATERIAL**
NEUARTIGE SMEKTISCHE PHASE EINES FLÜSSIGKRISTALLMATERIALS
NOUVEAU MATÉRIAU À CRISTAUX LIQUIDES EN PHASE SMECTIQUE A

(30) Priority: 10.09.2012 CN 201210331994
(43) Date of publication of application: 15.07.2015
(73) Proprietor: Halation Photonics Corporation, Jiangsu 215123 (CN)
(72) Inventor: LI, Wenlei, Suzhou Jiangsu 215123 (CN); SUN, Gang, Suzhou Jiangsu 215123 (CN); YIN, Huan, Suzhou Jiangsu 215123 (CN); TAN, Zhixian, Suzhou Jiangsu 215123 (CN); ZHAI, Huaibin, Suzhou Jiangsu 215123 (CN)
(74) Representative: AWA Sweden AB
(86) International application number: PCT/CN2012/082167
(87) International publication number: WO 2014/036766

(56) References cited:
- WO-A1-03/040074
- DE-A1- 4 409 431
- DE-A1- 19 934 798
- JP-A- H06 228 560
- JP-A- H08 325 174
- US-A- 4 853 150
- US-A- 4 921 632
- US-A1- 2011 075 074
- US-B1- 6 245 258

## Description

### Field of the Invention

The present invention relates to a smectic A phase liquid crystal material, which belongs to the field of optical display materials.

### Background

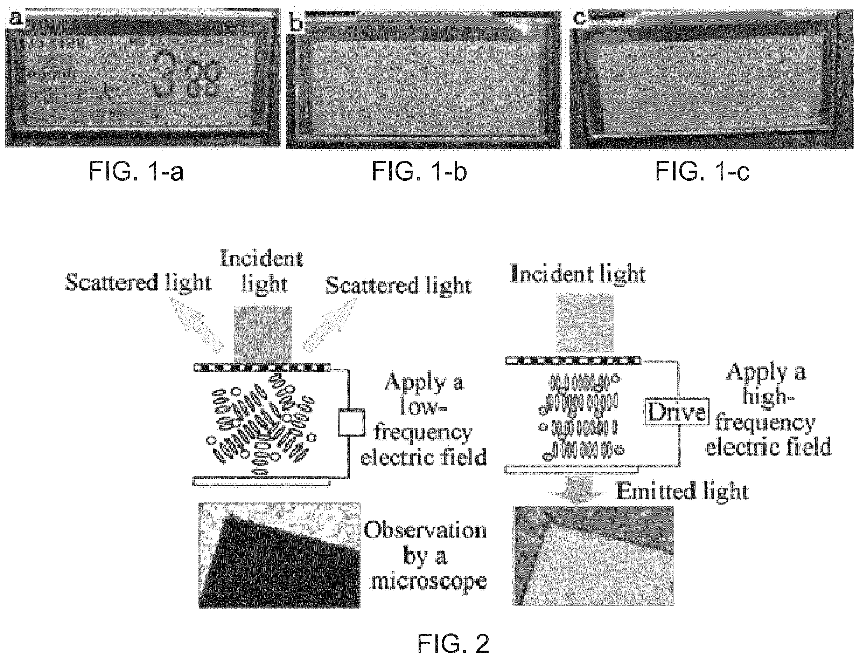

According to the phases, liquid crystal is classified into a nematic phase, a smectic phase and a cholesteric phase. The smectic phase is further classified into the smectic A, B, C, D, E, F, G, H, I, G phases and so on. The nematic liquid crystal and the cholesteric phase have a low viscosity and significant liquidity, while the smectic liquid crystal has a high viscosity. All the liquid crystals have the dielectric anisotropy, and the liquid crystal molecules can be driven by an electric field. After being driven by an electric signal, the molecular arrangement of the liquid crystal molecules having a low viscosity cannot be maintained and can be restored to the original arrangement after the electric signal is cut off. However, after being driven by an electric signal, the molecular arrangement of the liquid crystal molecules having a high viscosity can be maintained, but cannot be restored to the original arrangement, demonstrating a memory effect. Therefore, when being used in displaying, after the electric signal is removed, the smectic liquid crystal can maintain the display content and has a memory effect. As shown in FIG. 1-a, 1-b and 1-c, FIG. 1-a shows a state that an image is displayed, FIG. 1-b shows a state that the displayed image cannot be erased completely, leaving residual images, and FIG. 1-c shows a state that the image can be completely erased.

In 1978, in the article "Electrically induced scattering textures in smectic A phases and their electrical reversal", D. Coates et al. describes photoelectric driving characteristics of a smectic A phase compound 8CB. At that time, when being used as the smectic A phase liquid crystal display, the smectic A phase material such as 8CB, 8OCB has obvious disadvantageous of high drive voltage (generally about 200 V), a period of "aging" time required before use for steady driving, and a narrow range of temperature (about 10°C) for driving.

In Patent GB2274649A of Dow Corning Corp. in 1994, it is found that a siloxane smectic A phase liquid crystal material can lower the drive voltage (about 70 to 100 V), can work stably without being "aged" for a long period of time and had a broaden temperature range for driving. In WO2010/070606 of Cambridge Enterprise, this type of siloxane liquid crystal materials are used to be mixed with a nematic phase formulation to provide a wide-temperature-range smectic A phase material, which further extends the use temperature of the smectic A phase materials. In WO 2011/115611 A1 of Cambridge Enterprise, it is also proposed that the siloxane polymers are used in mixing of a smectic A phase liquid crystal formulation. The siloxane smectic A phase materials have the smectic A phase, and can induce other non-smectic A phase materials such that the mixed materials obtained by mixing with siloxane liquid crystal materials to have the smectic A phase. Therefore, the siloxane smectic A phase liquid crystal materials are a type of important materials in the smectic A phase formulation.

However, the siloxane smectic A phase liquid crystal has a strong memory effect, so that the displayed image cannot be completely erased, leaving residual images (as shown in FIG. 1b). In addition, since the birefringence of the siloxane smectic A phase materials is very low (Δn≈0.08), and the amount of the siloxane liquid crystal in the formulation is high, the smectic A phase liquid crystal formulation with siloxane as the main body has a low birefringence. In the scattering display mode, the contrast is associated with the birefringence of the liquid crystal material, where the higher the birefringence is, the higher the contrast is. Therefore, when the siloxane smectic A phase materials are used in display, the contrast is generally low.

US 6 245 258 B1 discloses a liquid crystal optical element in which a smectic liquid crystal material is used. The liquid crystal optical element is used in a display for displaying characters, figures and the like, a dimmer in which the transmission quantity of an incident light changes an optical shutter and the like.

DE 199 34 798 A1 discloses a liquid crystal control or display device that contains a chiral smectic liquid crystal mixture. The ratio DELTA / THETA of the angle between the rubbing direction and the smectic layer normal to the tilt angle is at least 0.41. The liquid crystal mixture preferably has the phase sequence I-N-C and the tilt angle THETA at 25 DEG C ranges from 19 DEG to 39 DEG.

US 4 853 150 A discloses novel organic compounds possessing liquid-crystal properties and, more particularly, 2-(4,3-disubstituted phenyl)-5-alkyl-1,3,2-dioxaborinane derivatives as components of a liquid crystal material and a liquid crystal material for use in electrooptical devices.

US 2011/075074 A1 discloses a liquid crystal composition and, in particular, methods, systems and devices for a liquid crystal mixture that exhibits blue phase and is intended for a polymer stabilized composite and a liquid crystal display comprising the same.

JP H06 228560 A discloses a smectic A liquid crystal composition and an optical switching element using the smectic A-liquid crystal composition.

WO 03/044074 A1 discloses smectic A liquid crystal compounds, liquid crystal mixtures comprising such compounds and liquid crystal devices thereof.

US 4 921 632 A discloses liquid crystal compounds and compositions showing a chiral smectic phase near the room temperature and/or having improved value of spontaneous polarization.

JP H08325174 A discloses a liquid crystal compound, an electro-optical display material, and a liquid crystal composition having a suitable physical properties using said liquid crystal compound.

DE 44 09 431 A1 discloses cyanophenylpyri(midine) derivatives, liquid crystal (LC) media comprising such derivatives and electro-optical devices thereof.

### SUMMARY OF THE INVENTION

The present invention is directed to a smectic A phase liquid crystal material, which can eliminate residual images of a smectic A phase formulation system of a siloxane liquid crystal and improve the contrast.

The present invention provides a smectic phase-inducing material. Based on this, the present invention further discloses mixing of the liquid crystals. This type of smectic A phase liquid crystal formulations have no residual images, have a high contrast, and the drive voltage is relatively low (about 20 to 50 V).

A smectic A phase liquid crystal material comprises at least one heterocyclic compound of Formula (III), (IV), (V), (VI) or (VII):. 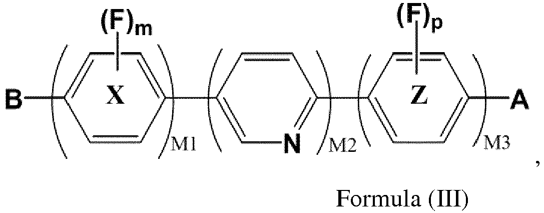 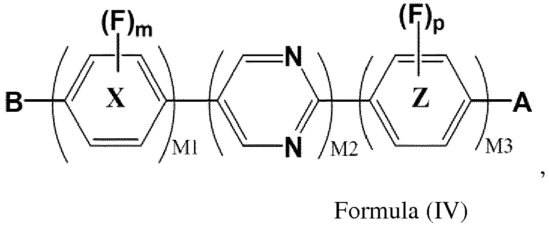 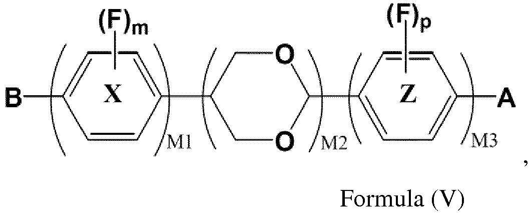 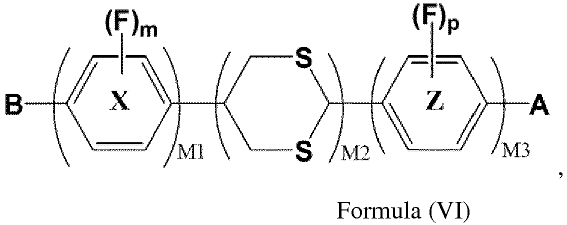 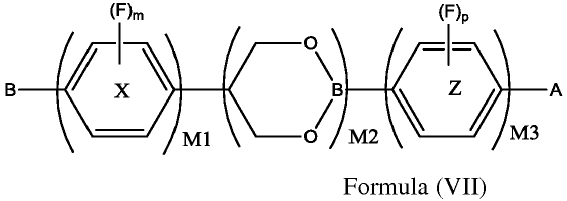
wherein for formulas (III)-(VII),m and p are independently 0, 1, 2, 3, or 4, M2 is 1 or 2, and M3 is 1 or 2;
X and Z are substituted or unsubstitued phenyl rings,
F is a fluorine atom, substituting any one or more of hydrogen atoms on the phenyl ring of X or Z; and
for formulas (III)-(IV) and (VI)-(VII), M1 is 0, 1 or 2; and
for formula (V), M1 is 1 or 2; and
for formula (III), B is selected from the group consisting of: CN, F, NCS, NCO, CF₃, CHF₂, CH₂F, OCF₃, OCHF₂, OCH₂F, NO₂, Cl, CH=CF₂ and OCH=CF₂; C1-C20 alkyl, C2-C20 alkenyloxy, C1-C20 silanyl and C1-C20 siloxanyl, and the halogenated groups thereof; and A is selected from the group consisting of CN, F, NCS, NCO, CF3, CHF2, CH2F, OCF3, OCHF2, OCH2F, NO2, Cl, CH=CF2 and OCH=CF2; C1-C20 alkyl, C2-C20 alkenyl, C2-C20 alkenyloxy, C1-C20 silanyl and C1-C20 siloxanyl, and the halogenated groups thereof;
for formula (IV), B is selected from the group consisting of: CN, F, NCS, NCO, CF3, CHF2, CH2F, OCF3, OCHF2, OCH2F, NO2, Cl, CH=CF2 and OCH=CF2; C2-C20 alkenyloxy, C1-C20 silanyl and C1-C20 siloxanyl, and the halogenated groups thereof; and A is selected from the group consisting of: F, NCS, NCO, CF3, CHF2, CH2F, OCF3, OCHF2, OCH2F, NO2, Cl, CH=CF2 and OCH=CF2; C2-C20 alkenyl, C2-C20 alkenyloxy, C1-C20 silanyl and C1-C20 siloxanyl, and the halogenated groups thereof; and
for formula (V), B is selected from the group consisting of: CN, F, NCS, NCO, CF3, CHF2, CH2F, OCF3, OCHF2, OCH2F, NO2, Cl, CH=CF2 and OCH=CF2; C1-C20 alkyl, C1-C20 alkoxy, C2-C20 alkenyloxy, C1-C20 silanyl and C1-C20 siloxanyl, and the halogenated groups thereof; and A is selected from the group consisting of: CN, F, NCO, CF3, CHF2, CH2F, OCF3, OCHF2, OCH2F, NO2, Cl, CH=CF2 and OCH=CF2; C1-C20 alkyl, C2-C20 alkenyl, C2-C20 alkenyloxy, C1-C20 silanyl and C1-C20 siloxanyl, and the halogenated groups thereof; and
for formula (VI), B is selected from the group consisting of: CN, F, NCS, NCO, CF3, CHF2, CH2F, OCF3, OCHF2, OCH2F, NO2, Cl, CH=CF2 and OCH=CF2; C1-C20 alkyl, C2-C20 alkenyloxy, C1-C20 silanyl and C1-C20 siloxanyl, and the halogenated groups thereof; and A is selected from the group consisting of: F, NCS, NCO, CF3, CHF2, CH2F, OCF3, OCHF2, OCH2F, NO2, Cl, CH=CF2 and OCH=CF2; C1-C20 alkyl, C2-C20 alkenyl, C2-C20 alkenyloxy, C1-C20 silanyl and C1-C20 siloxanyl, and the halogenated groups thereof; and
for formula (VII), B is selected from the group consisting of: CN, F, NCS, NCO, CF3, CHF2, CH2F, OCF3, OCHF2, OCH2F, NO2, Cl, CH=CF2 and OCH=CF2; C1-C20 alkyl, C1-C20 alkoxy, C2-C20 alkeny, C2-C20 alkenyloxy, C1-C20 silanyl and C1-C20 siloxanyl, and the halogenated groups thereof; and A is selected from the group consisting of: F, NCS, NCO, CF3, CHF2, CH2F, OCF3, OCHF2, OCH2F, NO2, Cl and OCH=CF2; C2-C20 alkenyl, C2-C20 alkenyloxy, C1-C20 silanyl and C1-C20 siloxanyl; and wherein the smectic A phase liquid crystal material further comprises at least one ester compound of Formula (II): 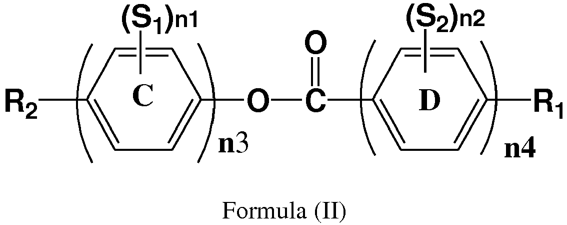
   wherein n₁ and n₂ are independently 0, 1, 2, 3, or 4,
   n₃ is 0, 1, or 2, and n4 is 0, 1, 2, or 3;
   C and D are independently substituted and unsubstitued phenyl rings;
   S₁ and S₂ are independently a N atom or a F atom, replacing any one or more of C atoms on the phenyl ring when S₁ or S₂ is a N atom, and replacing any one or more of H atoms on the phenyl ring when S₁ or S₂ is a F atom;
   R₁ and R₂ are independently selected from the group consisting of: F, NCS, NCO, CF₃, CHF₂, CH₂F, OCF₃, OCHF₂, OCH₂F, NO₂, Cl, CH=CF₂ and OCH=CF₂; C1-C20 alkyl, C1-C20 alkoxy, C1-C20 alkenyl, C1-C20 alkenyloxy, C1-C20 silanyl and C1-C20 siloxanyl, and the halogenated groups thereof; C1-C20 alkyl, C1-C20 alkoxy, C1-C20 alkenyl, C1-C20 alkenyloxy and isomers thereof with any -CH₂- substituted with -O-, -S-, -CF₂-, -CF₂O-, -CO-, -O-COO-, -CF=CF-, -CH=CF-, -CF=CH- and -CH=CH-.

The smectic A phase liquid crystal materials may further comprise at least one ionic compound. In the smectic A phase liquid crystal formulation of the present invention, an ionic compound may be added.

In The smectic A phase liquid crystal material of the present invention, the content of the compound of Formula (III), (IV), (V), (VI) or (VII), and (II) is 1 wt% to 100 wt%, preferably 10 wt% to 100 wt%, based on the total weight of the mixed liquid crystal layer; and the content of the ionic compounds is 0.0001 wt% to 10 wt%, preferably 0.0001 wt% to 1 wt%, based on the total weight of the mixed liquid crystal layer.

In the smectic A phase liquid crystal formulation of the present invention, in addition to the heterocyclic liquid crystal material of Formula (III), (IV), (V), (VI) or (VII) and the ester liquid crystal material of Formula (II), a nematic phase liquid crystal formulation, nematic liquid crystal compounds, other liquid crystal compounds or rod-like compounds having no liquid crystal properties may be added.

In addition, a dichromatic dye, a UV glue and similar materials may be further added to the smectic A phase liquid crystal material.

In the following, classification and preference of the ester compound of Formula (II) and the ionic compound in the smectic A phase liquid crystal formulation of the present invention, are further described in detail.

### 1. Ester compound

(1) The ester liquid crystal compound of Formula (II) may be a compound having a phenyl ring attached adjacently to the ester linkage and having a structure of Formula (VIII). 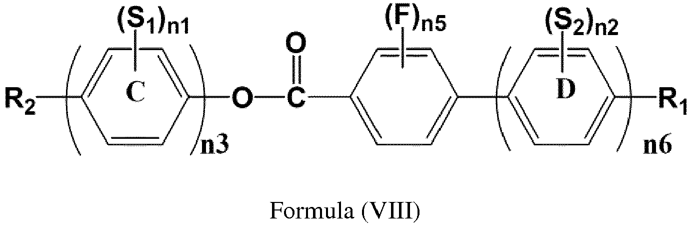
   In Formula (VIII), F represents a fluorine atom, n₅ is 0, 1, 2, 3, or 4, indicating that the hydrogen atoms on the phenyl ring adjacent to the ester linkage may be unsubstituted or substituted with fluorine, n₆ is 0, 1, or 2; and other letters R₁, R₂, S₁, S₂, C, D, F, n₁, n₂ and n₃ have the same meaning as that in Formula (II).
   (i) The ester liquid crystal compound of Formula (VIII) may have a structure of Formula (IX), wherein C and D are independently substituted or unsubstituted phenyl rings, S₁ is an N atom and may replace any C atom on the phenyl ring, and S₂ is an F atom and may replace any H atom on the phenyl ring. 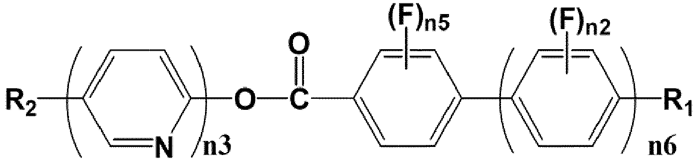 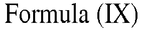
      In Formula (IX), letters have the same meaning as that in Formula (VIII).
   (ii) The ester liquid crystal compound of Formula (VIII) may have a structure of Formula (X), where C and D are independently substituted or unsubstituted phenyl rings, and S₁ and S₂ are an F atom and may replace any H atom on the phenyl ring. 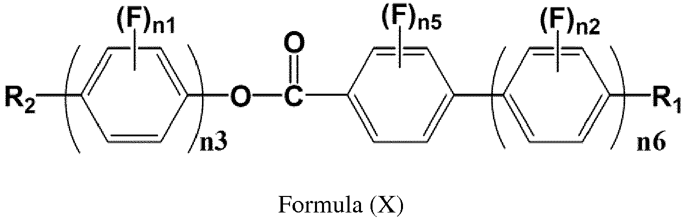
      In Formula (X), letters have the same meaning as that in Formula (VIII).
   (iii) The ester liquid crystal compound of Formula (VIII) may have a structure of Formula (XI), wherein C and D are phenyl ring, and S₁ and S₂ are an N atom and may replace any C atom on the phenyl ring. 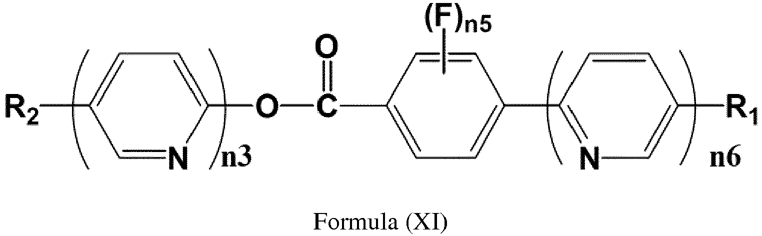
      In Formula (XI), letters have the same meaning as that in Formula (VIII).
   (iv) The ester liquid crystal compound of Formula (VIII) may have a structure of Formula (XII), where C and D are independently substituted or unsubstituted phenyl rings, S₁ is an F atom and may replace any H atom on the phenyl ring, and S₂ is an N atom and may replace any C atom on the phenyl ring. 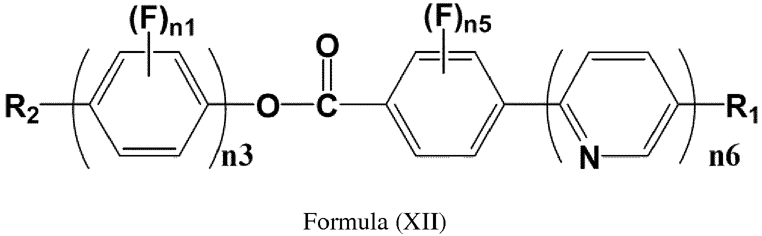
      In Formula (XII), letters have the same meaning as that in Formula (VIII).
(2) The ester liquid crystal compound of Formula (II) may have a compound having a pyridine ring attached adjacently to an ester linkage and having a structure of Formula (XIII) or (XIV).

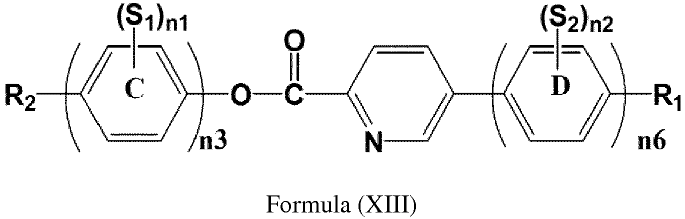

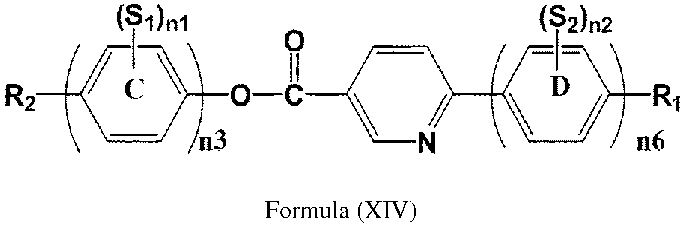

In Formula (XIII) or (XIV), letters have the same meaning as that in Formula (VIII).
(i) The ester liquid crystal compound of Formula (XIII) or (XIV) may have a structure of Formula (XV) or (XVI), where C and D are independently substituted or unsubstituted phenyl rings, S₁ is an N atom and may replace any C atom on the phenyl ring, and S₂ is an F atom and may replace any H atom on the phenyl ring. 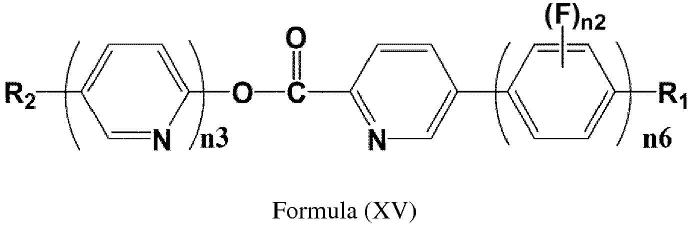 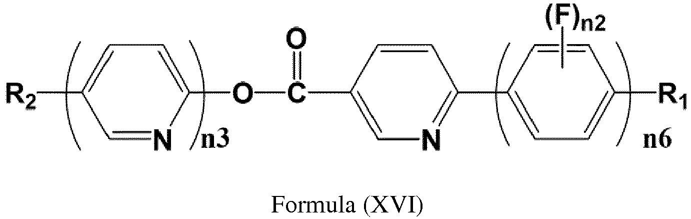
   In Formula (XV) or (XVI), letters have the same meaning as that in Formula (VIII).
(ii) The ester liquid crystal compound of Formula (XIII) or (XIV) may have a structure of Formula (XVII) or (XVIII), where C and D are independently substituted or unsubstituted phenyl rings, and S₁ and S₂ are an F atom and may replace any H atom on the phenyl ring. 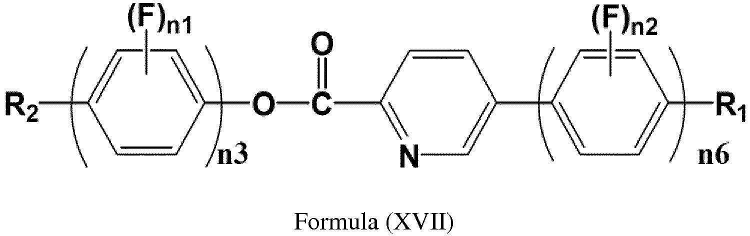 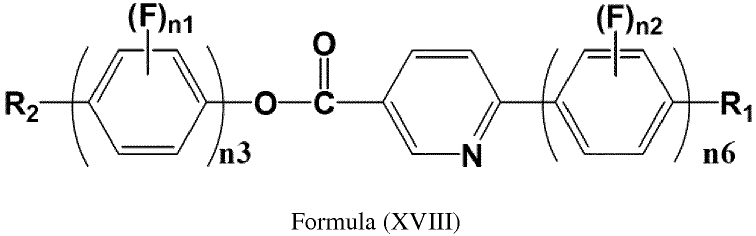
   In Formula (XVII) or (XVIII), letters have the same meaning as that in Formula (VIII).
(ii) The ester liquid crystal compound of Formula (XIII) or (XIV) may have a structure of Formula (XIX) or (XX), where C and D are independently substituted or unsubstituted phenyl rings, and S₁ and S₂ are an N atom and may replace any C atom on the phenyl ring. 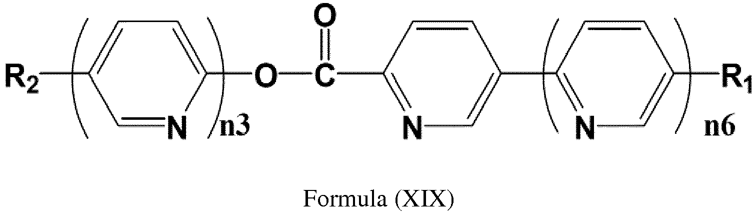 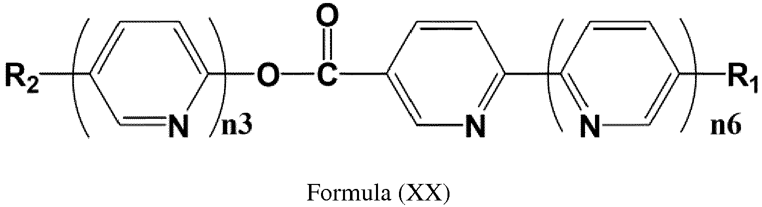
   In Formula (XIX) or (XX), letters have the same meaning as that in Formula (VIII).
(iv) The ester liquid crystal compound of Formula (XIII) or (XIV) may have a structure of Formula (XXI) or (XXII), where C and D are independently substituted or unsubstituted phenyl rings, S₁ is an F atom and may replace any H atom on the phenyl ring, and S₂ is an N atom and may replace any C atom on the phenyl ring.

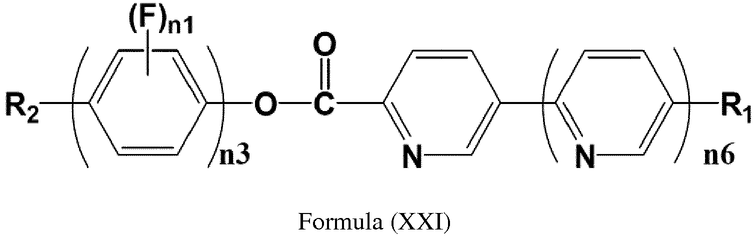

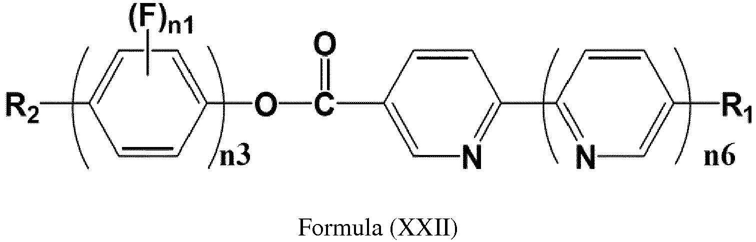

In Formula (XXI) or (XXII), letters have the same meaning as that in Formula (VIII).

### 2. Ionic compound

In the smectic A phase liquid crystal formulation of the present invention, the ionic compound may be selected from: sodium laurylsulfate, ethyl triphenylphosphonium iodide, (ferrocenylmethyl)trimethylammonium iodide, 1,2-dimethyl-3-butylimidazole hexafluorophosphate, tetraethylamine para-toluenesulfonate, phenyltriethylammonium iodide, 1-octyl-3-methylimidazole hexafluorophosphate, bis(tetra-n-butylamine)bis(1,3-dithiole-2-thione-4,5-dithiol)palladium (II), tetra-n-butyl bis(1,3-dithiole-2-thione-4,5-dithiol)nickel (III), bis(tetra-n-butylammonium) bis(1,3-dithiole-2-thione-4,5-dithiol)zinc, bis(tetra-n-butylammonium)tetracyanodiphenoquinodimethane, tetra-n-butylammonium bromide, cetylammonium perchlorate, cetyltetraammonium bromide, 1-butyl-3-methylimidazole tetrachloride ferrate, methyltriphenylphosphonium iodide, tetraphenylphosphonium iodide, cetyltrimethyl ammonium bromide, cetyltrimethyl ammonium perchlorate, cetyldimethylbenzylammonium chloride, dodecyl pyridine bromide, cetyl pyridine bromide, cetyl pyridine chloride and cetyltributylammonium bromide.

The smectic A phase liquid crystal material according to the present invention may be used in a smectic liquid crystal display device or a dimming device, especially in a device with a dual-frequency drive mode of low-frequency frosting and high-frequency clearing.

### BRIEF DESCRIPTION OF THE DRAWINGS

FIG. 1-a, 1-b and 1-c show several different states of a smectic A phase liquid crystal display screen respectively, where FIG. 1-a shows a state that an image is displayed, FIG. 1-b shows a state that the displayed image cannot be erased and residual images are remained, and FIG. 1-c shows a state that the image can be completely erased.
FIG. 2 is a schematic view of the drive display principle of a smectic liquid crystal.
FIG. 3-a, FIG. 3-b and FIG. 3-c show different working principles of several typical smectic A phases, where FIG. 3-a shows a first generation smectic A phase material, with 8CB as representative, FIG. 3-b shows a second generation smectic A phase materials, with a siloxane liquid crystal as representative, and FIG. 3-c shows a new generation (third generation) smectic A phase material, with a heterocyclic liquid crystal as representative.
FIG. 4 shows a 2.8-inch hollow liquid crystal cell.
FIG. 5 shows a 2.8-inch screen filled with a liquid crystal and sealed and bonded to IC.
FIG. 6 shows a drive test system of a 2.8-inch screen.
FIG. 7 is a schematic view of an instrument for testing the contrast by a microscopy method.
FIG. 8 shows a dimming glass that facilitates preparation of the present invention.

### DETAILED DESCRIPTION OF THE INVENTION

The drive display principle of the smectic phase liquid crystal applied to display is shown in FIG. 2. The smectic liquid crystal display screen generally includes an upper base plate and a lower base plate coated with an electrode layered structure and a mixed smectic liquid crystal sandwiched between the upper base plate and the lower base plate, and the mixed liquid crystal layer is generally formed by mixing a smectic liquid crystal, a conductive material, a spacer and sometimes a polymer. A capacitor structure formed by crossed electrodes from the upper and lower base plates is connected to an external drive circuit so the capacitor can apply electric energy to the mixed liquid crystal layer between the base plates, wherein the applied waveform may be high-frequency drive pulse for clearing operation and low-frequency drive pulse for frosting operation.

In a low frequency electric field (≤100 Hz), long-chain conductive ions (the conductive material, such as added organic conductive ions, such as tetrabutylammonium bromide, sodium laurylsulfate, cetyltrimethyl ammonium perchlorate, and tetraphenylphosphonium iodide) begin to move back and forth under the electric field force, thereby agitating and disturbing the smectic layer in ordered arrangement. This behavior is similar to the dynamic scattering effect of the nematic liquid crystal, and difference lies in that, the vortex plane formed during the smectic dynamic scattering is perpendicular to the direction of the applied electric field, while the vortex plane formed during the dynamic scattering of the nematic liquid crystals is parallel to the direction of the applied electric field. The molecular arrangement of the smectic liquid crystal is in a disordered state as shown in the left side in the FIG. 2 due to the high viscosity of the smectic liquid crystal. At this time, when observing the liquid crystal cell by using the transmittance light from a microscope, it can be viewed that the electrode region is in a back state that shads light, resulting in a frosting state.

In high-frequency electric field (≥1000 Hz), the organic conductive ions move back and forth slightly, and the agitation effect on the liquid crystal is negligible. Herein, under the electric field force, the major axis of the liquid crystal molecules are oriented in parallel to the direction of the electric field; when the electric signal is stopped, this regular arrangement is maintained, as shown in the right side in FIG. 2. At this time, when observing the liquid crystal cell by using the transmittance light from a microscope, it can be viewed that the electrode region is in a bright state that light can transmit, resulting in a clearing state.

The molecular arrangement of the smectic liquid crystal can also be remained in various states where the light transmittance is different, so as to achieve display at different grey levels. Therefore, the smectic liquid crystal has multi-stable characteristics.

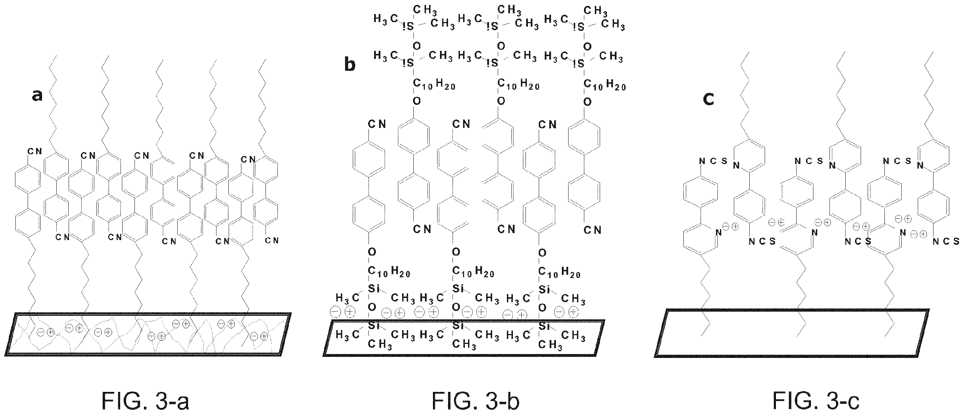

Difference in characteristics and working principle of several types of smectic A phase materials are analyzed (as shown in FIG. 3).

When the smectic A phase materials are subject to clearing, the liquid crystal molecules are arranged in the direction of the electric field due to dielectric anisotropy of liquid crystals itself. The liquid crystal molecules are disarranged by ions to cause frost, because different types of smectic A phase ions have different disarrangement mechanisms. Therefore, the principles of operation for different smectic A phase materials are differed in frosting mechanism.

FIG. 3-a shows a first generation smectic A phase material with 8CB as representative, which has a requirement on the electrode surface, and requires that the electrode surface needs to be rough, and needs to be driven and "aged" by a high initial voltage for a period of time for steady driving. Ions need to move in the smectic layer to exert the disturbing function, that is, the movement direction is perpendicular to the direction of an applied electric field, which requires that the conductivity (σ⊥) in the direction of the liquid crystal material perpendicular to molecular direction is greater than the conductivity (σ∥) in the direction parallel to the molecular direction. 8CB has a σ∥/σ⊥ value in the range of 0.5 to 1, and a not great conductivity in the direction perpendicular to the molecular direction, while the rough electrode surface allow the generation of an electric field perpendicular to the direction electric field, between the adjacent rough surfaces. Therefore, in low-frequency driving, ions first move in the smectic layer adjacent to the rough electrode surface, and thereby gradually driving other liquid crystal layers to move together, thereby finally reaching the frosting state.

FIG. 3-b shows a second generation smectic A phase material, with a siloxane liquid crystal as representative, which has no requirement on the electrode surface, and an electrode having a smooth surface can be used, and no aging is required for steady driving. The reason is that after the siloxanyl is introduced, this type of materials has a greater conductivity in the direction perpendicular to the molecular direction, and the σ∥/σ⊥ value is about 0.03. The ion additive is enriched in the siloxane layer, that is, between the adjacent smectic layers, and when electricity is applied, electrons move in the direction perpendicular to the electric field direction and between the smectic layers, thereby disturbing the liquid crystal layer to reach the frosting state.

FIG. 3-c shows a new generation (third generation) smectic A phase material, with a heterocyclic liquid crystal as representative, which also has no requirement on the electrode surface, an electrode having a smooth surface can be used, and no aging is required for steady driving. Due to the introduction of a heteroatom into the rigid nuclear moiety of the molecule, the conductivity of this type of materials in the perpendicular direction is increased. Ions are enriched around the heterocyclic ring, that is, in the smectic layer, and when electricity is applied, the ions move in the direction perpendicular to the direction of the electric field from the center of the smectic layer, thereby disturbing the liquid crystal layer to reach the frosting state. Since the ions directly disturb the liquid crystal layer at the center of the smectic layer when being subjected to frosting, so the frosting can be achieved easily and the degree of disorder of the smectic layer is high, thereby overcoming the memory effect and removing the residual images, and providing a strong light scattering and a high contrast at the frosting state. In addition, the dielectric anisotropy Δε of the siloxane liquid crystal is generally as low as about 0.8, and the dielectric anisotropy Δε of heterocyclic liquid crystal is generally as high as 8. The higher Δε is, the faster the response of the liquid crystal to the electric field is.
Therefore, this type of materials has a drive voltage lower than that of the siloxane liquid crystal material.

The functions of components of The smectic A phase liquid crystal material formulation of the present invention are as follows.

The smectic A phase-inducing component: The heterocyclic liquid crystal of Formula (I) function to induce the smectic phase. A smectic liquid crystal is formed as a result of a lateral attractive force between liquid crystal molecules higher than a terminal attractive force between the liquid crystal molecules, and introduction of a heteroatom into the rigid moiety of the liquid crystal molecule may increase the lateral attractive force between the liquid crystal molecules, so when being the smectic A phase, this type of liquid crystals can easily induce other non-smectic A phase materials to be the smectic A phase.

A component that increases the conductivity (σ⊥) in the direction perpendicular to the molecular direction: The ester liquid crystal material of Formula (II) functions to increase σ⊥. The reason is that the lone paired electrons are enriched in an outer orbit of oxygen nucleus, oxygen atom incorporated into the heterocyclic system further interacts with a heteroatom to provide a high conductivity in the direction perpendicular to the molecular direction, and frosting is more easily achieved by addition of an ester liquid crystal.

Other components optionally added: A low-viscosity liquid crystal material (fluorine-containing liquid crystal or cyclohexyl ring liquid crystal) is added for a fast response; a liquid crystal material having a great birefringence (such as alkyne liquid crystal) is added for a high contrast; a dichromatic dye is added for color display; and a UV glue is added for reduction of the working viscosity and enhancement of the adhesion of hollow liquid crystal cells.

The smectic A phase liquid crystal material of the present invention may not contain the ester liquid crystal material of Formula (II), if The smectic A phase liquid crystal material of the present invention does not contain the ester liquid crystal material of Formula (II), the drive voltage is increased.

The heterocyclic liquid crystal material and the ester liquid crystal material used in the present invention may be obtained through common chemical synthesis or other commercial way, and the other auxiliary materials, such as the nematic formulation, other liquid crystal materials or organic ionic compounds, may be directly purchased from the market.

The processes for preparing the heterocyclic liquid crystal of Formula (I) and the ester liquid crystal materials of Formula (II) are exemplified as follows.

The representative monomers are listed in Table 1:

**Table 1 List of examples for synthesized compounds in Formula (I) and (II), wherein compounds L9 and L11 are comparative compounds.**

| | | | |
|---|---|---|---|
| 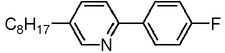 | L1 | 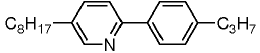 | L2 |
| 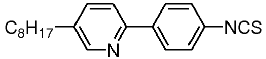 | L3 | 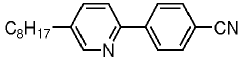 | L4 |
| 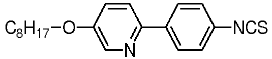 | L5 | 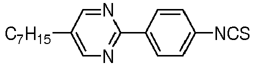 | L6 |
| 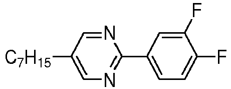 | L7 | 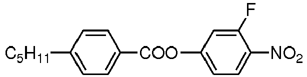 | L8 |
| 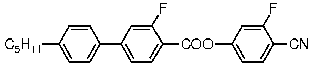 | L9 | 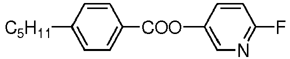 | L10 |
| 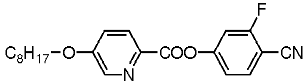 | L11 | | |

### Preparation of L1

### Preparation of L1 intermediate 1

In a three-necked round-bottomed flask, 23 g (1.1 eq) 1-bromo-n-heptane and 37g (1.0 eq.) triphenylphosphine are added in sequence, purged with nitrogen gas and then heated with stirring for 5 hours. The reaction liquid is filtered by suction, and the filter pad is washed in 200 ml x 3 ethyl acetate and dried to give 33 g white solid 1.

### Preparation of L1 intermediate 2

Under anhydrous and anaerobic conditions, in a four-necked rounded-bottom flask, 100 mL dry THF and 33 g (1.2 eq) intermediate 1 are added in sequence, and upon fall of temperature of the reaction system to -40°C, 35 ml n-butyl lithium is added dropwise; afterwards, the reaction system is warmed up to 0°C with stirring for 1 hour. Upon fall of temperature of the reaction system to -15°C, 11.6 g (1.2 eq) 2-bromo-5-aldo-pyridine is added dropwise, and then the reaction system is warmed up at refluxing with stirring for 3 hours. The reaction liquid is hydrolyzed, extracted with petroleum ether, and then was subject to column chromatography to give 11 g intermediate 2. [H-NMR (400M, CDCl₃) δ (ppm): 8.6 (s, 1H), 7-8 (d, 2H), 6.5 (d, 1H), 6.1 (m, 1H), 1.2-1.4 (m, 8H), 0.95 (m, 3H)].

### Preparation of L1 intermediate 3:

In a four-necked rounded-bottom flask, 50 mL anhydrous ethanol and 10 g (1.0 eq) intermediate 2 are added in sequence; the oxygen is removed from the reaction system, then palladium/carbon (0.1 g) is added, then H2 is fed; the reaction system is stirred at 40°C for 8 hours; the reaction liquid is filtered for removal of palladium/carbon and concentrated to give total of 10.0 g intermediate 3. [H-NMR (400M, CDCl₃) δ (ppm): 8.6 (s, 1H), 7-8 (d, 2H), 2.6 (m, 2H), 1.2-1.7 (m, 12H), 0.95 (m, 3H)].

### Preparation of L1

In a flask, 40 mL anhydrous ethanol, 40 mL deionized water, 13 g (2.5 eq) potassium carbonate, 10 g (1.0 eq) intermediate 3 and 6.2 g (1.2 eq) 4-fluorophenylboronic acid are added in sequence, the oxygen is removed from the reaction system, then 0.1 g Pd(dppf)₂Cl₂ is added; the reaction system is warmed up at refluxing, and the reaction is carried out for 8 hours; the reaction liquid is extracted with 100 mL ethyl acetate, and purified by column chromatography to give total of 9.0 g product. [H-NMR (400M, CDCl₃) δ (ppm): 8.6 (s, 1H), 7-8 (d, 6H), 2.6 (m, 2H), 1.2-1.7 (m, 12H), 0.95 (m, 3H)].

### Preparation of L2

The preparation process is same as that of L1, except that 4-propylphenylboronic acid is used as the raw material. L2 [H-NMR (400M, CDCl₃) δ (ppm): 8.6 (s, 1H), 7-8 (d, 6H), 2.6 (m, 4H), 1.2-1.7 (m, 14H), 0.95 (m, 6H)].

### Preparation of L4

The preparation process is same as that of L1, except that p-cyanophenylboronic acid is used as the raw material. L4 [H-NMR (400M,CDCl₃) δ (ppm): 8.6 (s,1H), 7-8 (d, 6H), 2.6 (m, 2H), 1.2-1.7 (m, 12H), 0.95(m, 3H)].

### Preparation of L3

The process the same as that in preparation of L1 intermediates is used to prepare L3 intermediate 1, L3 intermediate 2 and L3 intermediate 3.

### Preparation of L3 intermediate 4

In a flask, 20 mL anhydrous ethanol, 10 mL deionized water, 13 g (2.5 eq) potassium carbonate, 5.1 g (1.0 eq) intermediate 3 and 3.4 g (1.3 eq) 4-aminophenylboronic acid are added in sequence, the oxygen is removed from the reaction system, and then 0.05 g Pd(Ph₃P)₄ is added; the reaction system is warmed up at refluxing, and the reaction is carried out for 8 hours; the reaction liquid is purified by column chromatography to give 5.2 g product. [H-NMR (400M, CDCl₃) δ (ppm): 8.6 (s, 1H), 7-8 (d, 6H), 5.4 (s, 2H), 2.6 (m, 2H), 1.2-1.7 (m, 12H), 0.95 (m, 3H)].

### Preparation of L3

In a flask, 5.0 g (1.0 eq.) L3 intermediate 4 is dissolved in 30 mL dichloromethane; 15 mL water and 5.3 g (3 eq.) calcium carbonate are added; the reaction system is cooled to 0°C; 2.65 g (1.3 eq.) thiophosgene is added dropwise, and stirred for 6 hours at 0°C; the reaction liquid i purified by column chromatography to give 4.2 g product. [H-NMR (400M, CDCl₃) δ (ppm): 8.6 (s, 1H), 7-8 (d, 6H), 2.6 (m, 2H), 1.2-1.7 (m, 12H), 0.95 (m, 3H)].

### Preparation of L5

### Preparation of L5 intermediate 1

In a reaction system, the solvent DMF (dry), 5 g (1.0 eq.) 2-bromo-5-hydroxypridine, 6.1 g (1.1 eq.) 1-bromooctane, 9.9 g (2.5 eq.) potassium carbonate and 0.5 g (0.1 eq.) potassium iodide are added and reacted with stirring for 4 hours at 90°C; and the reaction liquid is purified to give 8.0 g product 1. [H-NMR (400M, CDCl₃) δ (ppm): 8.7 (s, 1H), 7-8 (d, 2H), 4.1 (m, 2H), 1.2-1.7 (m, 12H), 0.95 (m, 3H)].

### Preparation of L5 intermediate 2

In a reaction system, 30 mL anhydrous ethanol, 30 mL deionized water, 7.0 g (1.0 eq) L5 intermediate 1, 8.44 g (2.5 eq) potassium carbonate and 4.4 g (1.3 eq) 4-aminophenylboronic acid are added in sequence; the oxygen is removed from the reaction system; then 0.1 g Pd(Ph₃P)₄ is added; the reaction is warmed up at refluxing, and the reaction is carried out for 16 hours, and the reaction liquid is purified by column chromatography to give 6.5 g product 2. [H-NMR (400M, CDCl₃) δ (ppm): 8.5 (s, 1H), 7-8 (d, 6H), 5.1 (s, 2H), 4.0 (m, 2H), 1.2-1.7 (m, 12H), 0.95 (m, 3H)].

### Preparation of L5

In a flask, 6.0 g (1.0 eq.) L5 intermediate 2 is dissolved in 20 mL dichloromethane; 10 mL water and 6.0 g (3 eq.) calcium carbonate are added; the reaction system is cooled to 0°C; 3.0 g (1.3 eq.) thiophosgene is added dropwise, and stirred for 6 hours at 0°C; the reaction liquid is purified by column chromatography to give 5.5 g product. [H-NMR (400M, CDCl₃) δ(ppm): 8.5 (s, 1H), 7-8 (d, 6H), 3.9 (m, 2H), 1.2-1.7 (m, 12H), 0.95 (m, 3H)].

### Preparation of L6

### Preparation of L6 intermediate 1

In a flask, 82.8 g (1.2 eq.) potassium carbonate and 100 ml morpholine are added; a stir bar, a thermometer and a constant pressure dropping funnel are installed; the reaction system is cooled externally to0°C and less, and71 g (1.0 eq.) n-nonyl aldehyde is added dropwise; after complete addition for about 30 minutes, the reaction system is maintained for 8 hours at 0 to 5°C; the reaction liquid is filtered by suction; the funnel is flushed with 200 ml petroleum ether (90 to 105°C) several times, the filtrate is concentrated and distilled at reduced pressure, and the products of 94-96°C are collected to give 52 g colorless liquid product 1.

### Preparation of L6 intermediate 2

In a flask, 100 ml dichloromethane, 18 g (1.2 eq.) triethyl orthoformate and 21 g (1.0 eq.) L6 intermediate 1 are added. A stirring bar, a thermometer and a dropping funnel are installed, and the temperature is decreased to below -40°C; and then 17 g (1.2 eq.) solution of boron trifluoride in diethyl ether is added dropwise with the temperature being controlled to -40 to -45°C; after complete addition for about 1 hour, the reaction system is maintained for 3 hours at -40°C and naturally warmed up to 25°C, and a mixture of 3 g sulfuric acid, 57 ml water and 12 ml ethanol is added and hydrolyzed for 3 hours; after separation, the water layer is extracted twice with dichloromethane, and the organic phases are combined and washed with 5% aqueous potassium carbonate solution three times, and is washed with water to be neutral, and is dried over anhydrous magnesium sulfate and concentrated to give 19 g intermediate 2.

### Preparation of L6 intermediate 3

To a reaction system, 150 ml methanol and 2.5 g sodium wire are added, and an exothermic reaction is carried out at refluxing to give a sodium methoxide solution; after the reaction system is cooled to 25°C, 19.7 g (1.0 eq.) p-nitrobenzamidine hydrochloride and 28 g (1.5 eq.) L6 intermediate 2 are added, N₂ is fed for protection; after about 30-hour reaction at a temperature controlled to be 30°C, the reaction liquid is frozen at -10°C and is filtered by suction, and the cake is washed with a small amount of petroleum ether (cold) 3 times and washed with water 3 times, is dried by suction and then is recrystallized once in 3-fold ethanol to give 6 g L6 intermediate 3 as an off-white solid. [H-NMR (400M, CDCl₃) δ (ppm): 8.3 (s, 2H), 7-8 (d, 4H), 2.7(m, 2H), 1.2-1.7 (m, 10H), 0.95 (m, 3H)].

### Preparation of L6 intermediate 4

In a one-necked flask, 6 g (1.0 eq.) L6 intermediate 3, 2 g palladium/carbon and 200 ml ethanol are added, purged with nitrogen gas and heated to 60°C; 6 g (2.0 eq.) hydrazine hydrate is slowly added dropwise and refluxed for 1 hour; the reaction liquid is cooled to 25°C, and is filtered by suction; the filtrate is concentrated and recrystallized in 2-fold petroleum ether to give 4.5 g L6 intermediate 4 as an off-white solid. [H-NMR (400M, CDCl₃) δ (ppm): 8.3 (s, 2H), 7-8 (d, 4H), 5.6 (m, 2H), 2.7 (m, 2H), 1.2-1.7 (m, 10H), 0.95 (m, 3H)].

### Preparation of L6

In a three-necked flask, 4.5 g (1.0 eq.) L6 intermediate 4, 30 ml chloroform, 5 g (2.5 eq.) calcium carbonate and 20 ml deionized water are added at a temperature of 0°C, and 5 g (1.6 eq.) thiophosgene is added dropwise; and then the reaction system is stirred for 3 hours at a temperature controlled to be 0-5°C; the reaction system is filtered by suction, the cake is washed in 200 ml dichloromethane, and the filtrate is separated, concentrated and is subject to column chromatography to give 4 g white solid product. [H-NMR (400M, CDCl₃) δ (ppm): 8.3 (s, 2H), 7-8 (d,4H), 2.7 (m, 2H), 1.2-1.7 (m, 10H), 0.95 (m, 3H)].

### Preparation of L7

L7 intermediate 1, L7 intermediate 2 and L7 are prepared in the same manner as the former three processes for preparation of L6, except that after preparation of L7 intermediate 2, L7 intermediate 2 is immediately reacted to prepare L7, where the raw material is 3,4-difluoro benzamidine.

L7 final product [H-NMR (400M, CDCl₃) δ (ppm): 8.5 (s, 2H), 7-8 (d, 3H), 2.7 (m, 2H), 1.2-1.7 (m, 10H), 0.95 (m, 3H)].

Preparation of L8 (many synthetic processes are available for this type of monomers; herein convenient processes are exemplified):

To a reaction system, 120mL dichloromethane, 15g (1.0eq.) 4-pentylbenzoic acid, 12.5 g (1.02 eq.) 3-fluoro-4-nitrophenol, 16.9 g (1.05 eq.) DCC and a catalytic amount of DMAP (0.05 g) are added and stirred for 8 hours at 30°C for reaction, and then are processed to give 20 g product. [H-NMR (400M, CDCl₃) δ: (ppm) 7-8 (d, 7H), 2.5 (m, 2H), 1.2-1.7 (m, 8H), 0.95 (m, 3H)].

### Preparation of L9

The preparation method is the same as that of L8, except only that 2-fluoro-4-pentylbenzoic acid and 3-fluoro-4-cyanophenol are used. [H-NMR (400M, CDCl₃) δ: (ppm) 7-8 (d, 6H), 2.5(m, 2H), 1.2-1.7 (m, 8H), 0.95(m, 3H)].

### Preparation of L10

The preparation method is the same as that of L8, except that 2-fluoro-4-hydroxypridine is used. [H-NMR (400M, CDCl₃) δ: (ppm) 8.4 (s, 1H), 7-8 (d, 6H), 2.5 (m, 2H), 1.2-1.7 (m, 8H), 0.95 (m, 3H)].

### Preparation of L11

The preparation method is the same as that of L8, except that 2-fluoro-4-hydroxyphenol and 5-octyloxy-2-carboxylic acid pyridine are used. [H-NMR (400M, CDCl₃) δ: (ppm) 9.0 (s, 1H), 7-8.5 (d, 5H), 4.0(m, 2H), 1.2-1.7 (m, 12H), 0.95 (m, 3H)].

### Advantages of the present invention

1. By applying the new generation smectic A phase liquid crystal formulation material of the present invention to the existing smectic liquid crystal display device, the occurrence of residual images can be effectively avoided.
2. By applying the new generation smectic A phase liquid crystal formulation material of the present invention to the existing smectic liquid crystal display device, the contrast of the existing display device can be effective improved. The contrast acceptable for human eyes is generally 5:1, while the new generation smectic A phase liquid crystal formulation material of the present invention has a contrast higher than 10 without any optical processing aids, thereby providing good visual effects.
3. The drive voltage for the new generation smectic A phase liquid crystal formulation material of the present invention is low (20-50 V), thereby saving energy.

In the following, the present invention is further described with reference to the accompanying drawings and specific embodiments, which are not intended to limit the scope of the present invention.

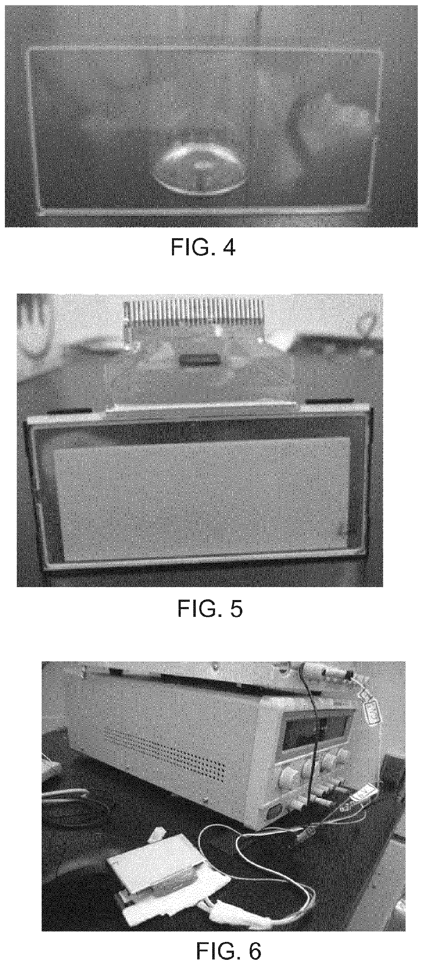

In the present invention, mixing and tests are performed following the following liquid crystal mixing experimental procedure:
1. first, liquid crystal materials are weighed at a specific ratio, and are added one by one into a glass vial;
2. the vial contained the materials is placed into an oven and heated until the liquid crystals is completely clear;
3. the liquid crystal is fully and uniformly mixed through ultrasonic shock or magnetic stirring;
4. the mixed liquid crystal is heated and filled in vacuum by a crystal filler into a hollow liquid crystal cell having a thickness of 12 µm thickness and a size of 2.8 inch, as shown in FIG. 4, where the hollow cell has a 12-µm spacer, a piece of upper glass and a piece of lower glass are provided with an ITO electrode on a surface facing the spacer, each piece of glass is provided with a 72-row and 184-conlumn ITO electrode, the junctions of the rows and columns are pixels, and the hollow cell is sealed with a sealant, but one site is remained unsealed and serves as a liquid crystal filling port.
5. after cooling, the liquid crystal cell is sealed with a UV glue, and then an integrated control circuit with an IC drive chip is bonded to electrodes of the-iquid crystal cell (as shown in FIG. 5).
6. the IC chip for the liquid crystal cell is connected to the drive circuit board, the liquid crystal cell is driven by an external power supply (that can supply an alternating voltage of 0 to 70 V), image scanning is performed at 2 KHz and frosting is performed at 30 Hz to erases the image. The whole device is shown in FIG. 6.
7. the liquid crystal cell was tested for the contrast.
8. the liquid crystal cell is driven cyclically (the image is scanned continuously at a high frequency, as shown in FIG. 1a; then the image is erased by low-frequency frosting, as shown in FIG. 1c); that is, after "aging" the image for a period of time or just leaving the image undriven at room temperature for a period of time, scanning and frosting operation are performed on the image of the liquid crystal cell, and whether residual images are present is determined through observation.

The contrast of the smectic liquid crystal display is a ratio of the light transmittance at the clearing state to the light transmittance at the frosting state, and in general, all materials have a substantially the same light transmittance at the clearing state; therefore, the contrast mainly depends on the light transmittance of the material at the frosting state, that is, the scattering state of the smectic liquid crystal material.

Since no standard method for testing the contrast of the reflective smectic liquid crystal display device is available in the industry, multiple universal and simple methods are used to test the contrast, and finally, a test method having a visual effect closer to that of human eye is selected and used as the standard of verification and comparison.

### Contrast test method: microscopy method

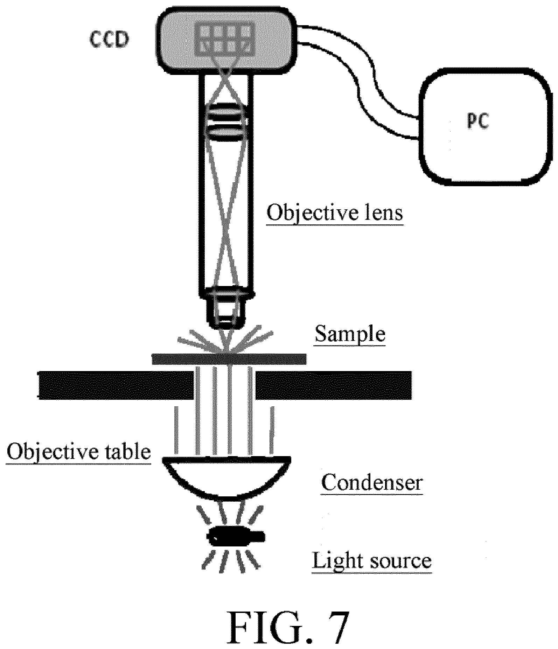

This test method is a simple method that is often used for testing and has measurement results close to human eyes, and the apparatus used by the method is shown in FIG. 7. In the microscopy method, a light transmittance measurement system HL-TT-MS is used as the contrast test apparatus, a DM_2500M metalloscope from Leica Corp. is used as the imaging device, an MV-VD120SC industrial CCD camera from Microvision Inc. is used as the optical signal collector, and HL-CR-11A software from Halation Photonics Co., Ltd. is used as the numerical calculation software.

### Test procedure:

1. A sample is placed on an objective table, and the focal length of the microscope is adjusted, so that the sample can be imaged clearly.
2. The sample was removed, and the HL-CR-11A software was used to compute with the numerical values at each points collected by CCD as follows: Yi=0.299*R+0.587*G+0.114*B.
3. The Yi values at each points are summed to give a normalized factor Y0=∑Yi.
4. The sample is placed onto the objective table, and the HL-CR-11A software is used to compute the Y value at this point Y=∑Yi.
5. The transmittance of the sample is defined as T=(Y/Y0)*100%.
6. For the smectic liquid crystal sample, the transmittance at clearing state Tc=(Yc/Y0)*100%, the transmittance at frosting state Ts=(Ys/Y0)*100%, and the contrast Cr=Tc/Ts.

In brief, first, in the situation that no liquid crystal cell is placed on the objective table and a light source (light ray emitted from a halide lamp equipped on the microscope) directly enter the objective lens, the light rays are collected by a receiver, the receiver converts the collected light rays into corresponding electric signals and send the electric signals to software of a computer, and the software records an electric signal B at this point as a basic reference value. Next, the luminance L of the light source at this point is fixed, the liquid crystal cell is placed on an objective table and the height of the objective table is adjusted, so that the liquid crystal cell can be clearly observed from an ocular lens; the receiver converts the light ray energy collected from the liquid crystal cells at the clearing state and the frosting state into an electric signal and sent the electrical signal to the computer. The computer software compares the light ray energy received at the clearing state and the frosting state with the basic reference. That is, the electric signal Q of the light ray energy received at the clearing state is divided by the basic reference value B to obtain a transmittance value QL at the clearing state, the electric signal M of the light ray energy received at the frosting state is divided by the basic reference value B to obtain a transmittance value ML at the frosting state, and the transmittance at the clearing state is divided by the transmittance at the frosting state to obtain a contrast value QL/ML*100%.

In the following embodiments, mixing is performed according to the crystal mixing method described in the present invention, and the contrast is tested.
Embodiment 1 Comparison between the nematic formulations respectively mixed with a siloxane liquid crystal material and a heterocyclic liquid crystal material 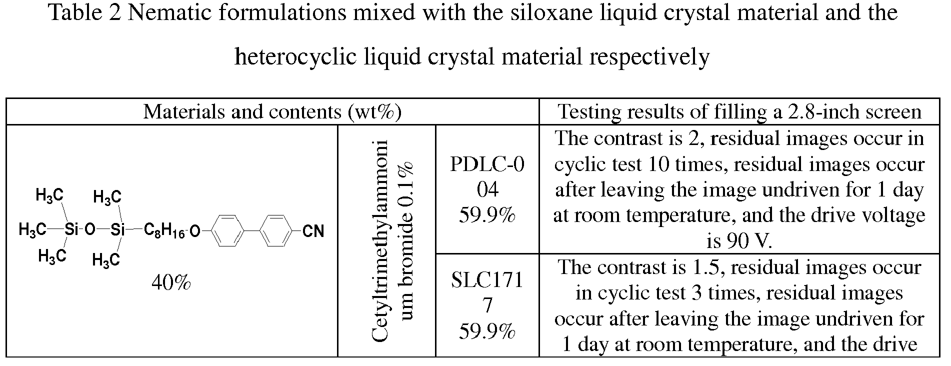 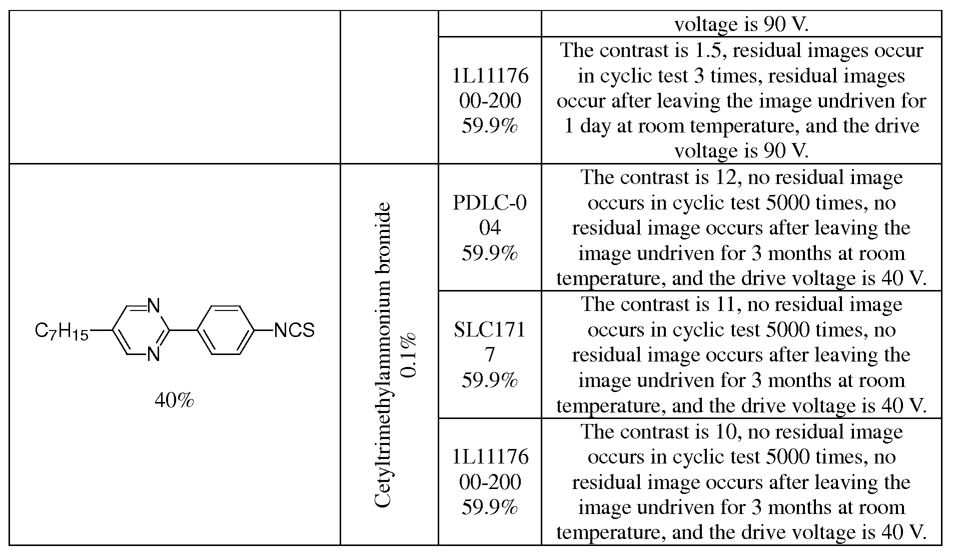
The mischcrystal was heated to be clear, filled in vacuum into a 2.8-inch screen, sealed and bonded, and the contrast was tested. In Embodiment 1, PDLC-004 was available from Shijiazhuang Huarui Technology Co.ltd, and SLC1717 and 1L1117600-200 were available from Shijiazhuang Yongshen Huaqing Liquid Crystal Co. Ltd.
Embodiment 2 Formulation and performance of a smectic A phase liquid crystal with a nitrogen-containing heterocyclic ring as the main body and added with any ester liquid crystal

**Table 3 Composition of the mischcrystal of Embodiment 2 (comparative)**

| Materials | Content, wt% |
|---|---|
| 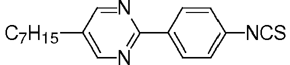 | 15 |
| 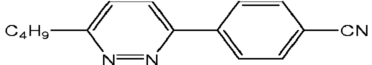 | 25 |
| 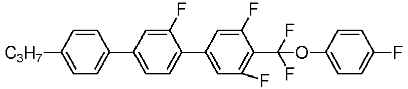 | 20 |
| 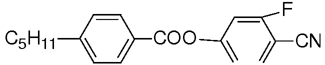 | 9.9 |
| 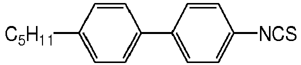 | 25 |
| 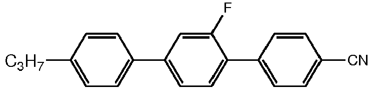 | 5 |
| Tetrabutylammonium bromide | 0.1 |

The mischcrystal was heated to be clear, filled in vacuum into a 2.8-inch screen, sealed and bonded. The contrast was tested to be 11:1, no residual image occurred after aging 5000 times, and the drive voltage was 30 to 40 V.
Embodiment 3 Formulation and performance of a smectic A phase liquid crystal with a pyridine heterocyclic ring as the main body and added with any ester liquid crystal

**Table 4 Composition of the mischcrystal of Embodiment 3 (comparative)**

| Materials | Content, wt% |
|---|---|
| 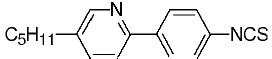 | 40 |
| 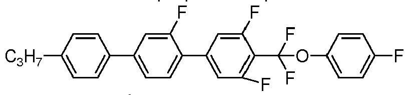 | 20 |
| 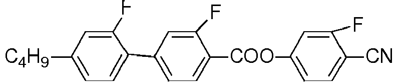 | 9.9 |
| 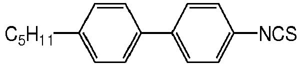 | 25 |
| 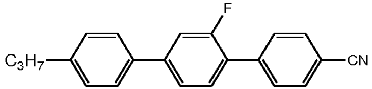 | 5 |
| Cetyltrimethylammonium bromide | 0.1 |

The mischcrystal was heated to be clear, filled in vacuum into a 2.8-inch screen, sealed and bonded. The contrast was tested to be 11:1, no residual image occurred after aging 5000 times, and the drive voltage was 30 to 40 V.
Embodiment 4 Formulation and performance of a smectic A phase liquid crystal with a pyrimidine heterocyclic ring as the main body and added with any ester liquid crystal

**Table 5 Composition of the mischcrystal of Embodiment 4 (comparative)**

| Materials | Content, wt% |
|---|---|
| 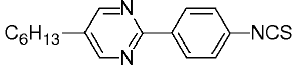 | 40 |
| 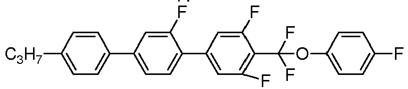 | 20 |
| 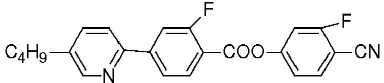 | 9.9 |
| 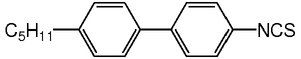 | 25 |
| 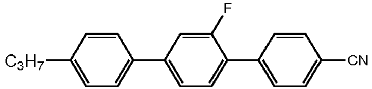 | 5 |
| Cetyltributylammonium bromide | 0.1 |

The mischcrystal was heated to be clear, filled in vacuum into a 2.8-inch screen, sealed and bonded. The contrast was tested to be 11:1, no residual image occurred after aging 5000 times, and the drive voltage was 30 to 40 V.
Embodiment 5 Formulation and performance of a smectic A phase liquid crystal with an oxygen heterocyclic ring as the main body and added with any ester liquid crystal

**Table 6 Composition of the mischcrystal of Embodiment 5 (comparative)**

| Materials | Content, wt% |
|---|---|
| 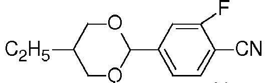 | 15 |
| 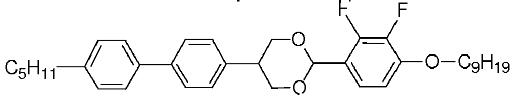 | 25 |
| 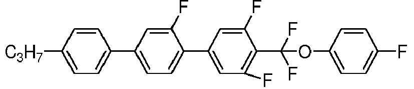 | 20 |
| 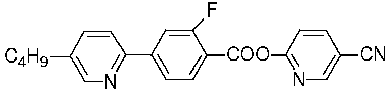 | 9.9 |
| 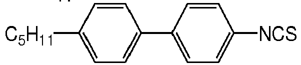 | 25 |
| 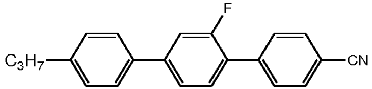 | 5 |
| Cetyltrimethylammonium perchlorate | 0.1 |

The mischcrystal was heated to be clear, filled in vacuum into a 2.8-inch screen, sealed and bonded. The contrast was tested to be 11:1, no residual image occurred after aging 5000 times, and the drive voltage was 30 to 40 V.
Embodiment 6 Formulation and performance of a smectic A phase liquid crystal with a sulfur-containing heterocyclic ring as the main body and added with any ester liquid crystal

**Table 7 Composition of the mischcrystal of Embodiment 6**

| Materials | Content, wt% |
|---|---|
| 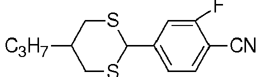 | 15 |
| 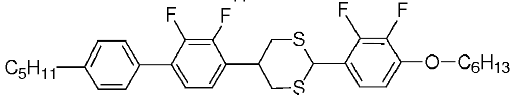 | 25 |
| 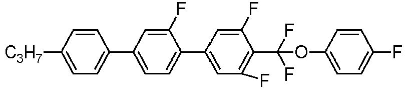 | 20 |
| 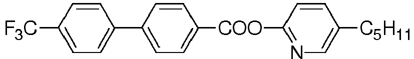 | 9.9 |
| 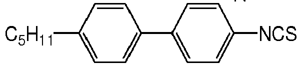 | 25 |
| 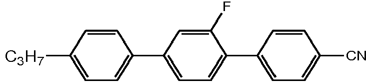 | 5 |
| Cetyltrimethylammonium perchlorate | 0.1 |

The mischcrystal was heated to be clear, filled in vacuum into a 2.8-inch screen, sealed and bonded. The contrast was tested to be 11:1, no residual image occurred after aging 5000 times, and the drive voltage was 30 to 40 V.
Embodiment 7 Formulation and performance of a smectic A phase liquid crystal with a boron-containing heterocyclic ring as the main body and added with any one or more ester liquid crystals

**Table 8 Composition of the mischcrystal of Embodiment 7 (comparative)**

| Materials | Content, wt% |
|---|---|
| 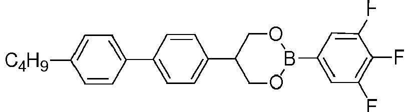 | 15 |
| 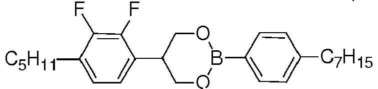 | 25 |
| 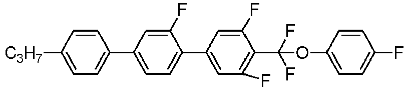 | 20 |
| 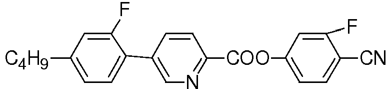 | 9.9 |
| 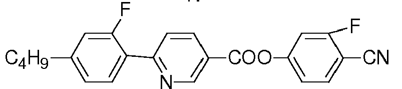 | 5 |
| 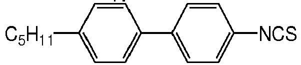 | 20 |
| 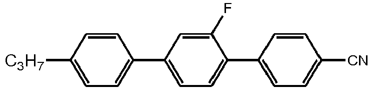 | 5 |
| Cetyltrimethylammonium perchlorate | 0.1 |

The mischcrystal was heated to be clear, filled in vacuum into a 2.8-inch screen, sealed and bonded. The contrast was tested to be 11:1, no residual image occurred after aging 5000 times, and the drive voltage was 30 to 40 V.
Embodiment 8 Formulation and performance of a smectic A phase liquid crystal with any combination of various heterocyclic rings as the main body and added with any one or more ester liquid crystals

**Table 9 Composition of the mischcrystal of Embodiment 8 (comparative)**

| Materials | Content, wt% |
|---|---|
| 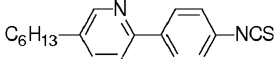 | 30 |
| 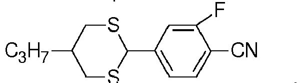 | 5 |
| 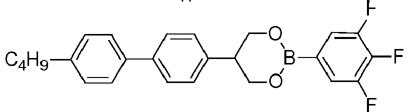 | 10 |
| 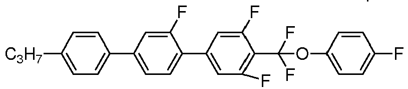 | 15 |
| 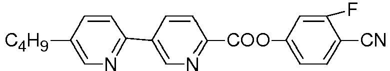 | 9.9 |
| 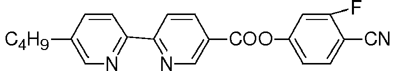 | 5 |
| 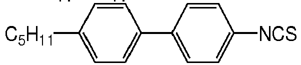 | 20 |
| 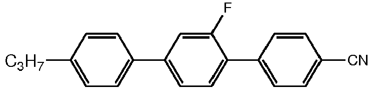 | 5 |
| Cetyltrimethylammonium perchlorate | 0.1 |

The mischcrystal was heated to be clear, filled in vacuum into a 2.8-inch screen, sealed and bonded. The contrast was tested to be 11:1, no residual image occurred after aging 5000 times, and the drive voltage was 30 to 40 V.
Embodiment 9 Formulation and performance of a smectic A phase liquid crystal with any combination of various heterocyclic rings as the main body and added with any one or more of ester liquid crystal

**Table 10 Composition of the mischcrystal of Embodiment 9 (comparative)**

| Materials | Content, wt% |
|---|---|
| 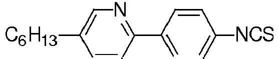 | 30 |
| 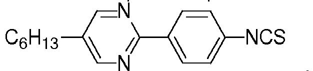 | 5 |
| 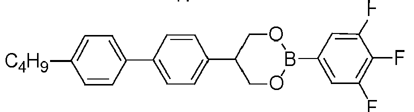 | 10 |
| 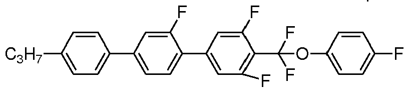 | 15 |
| 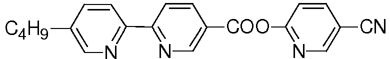 | 9.9 |
| 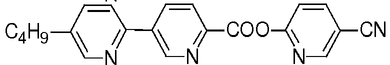 | 5 |
| 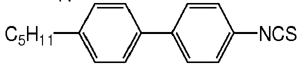 | 20 |
| 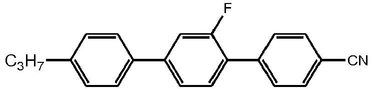 | 5 |
| Cetyltrimethylammonium perchlorate | 0.1 |

The mischcrystal was heated to be clear, filled in vacuum into a 2.8-inch screen, sealed and bonded. The contrast was tested to be 11:1, no residual image occurred after aging 5000 times, and the drive voltage was 30 to 40 V.
Embodiment 10 Formulation and performance of a smectic A phase liquid crystal obtained by introducing an alkyne liquid crystal material for improving the contrast into a formulation with a heterocyclic ring as the main body and added with any one or more ester liquid crystals

**Table 11 Composition of the mischcrystal of Embodiment 10**

| Materials | Content, wt% |
|---|---|
| 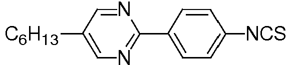 | 30 |
| 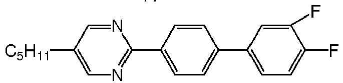 | 5 |
| 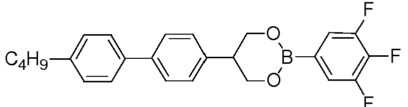 | 10 |
| 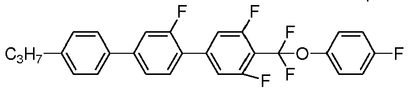 | 15 |
| 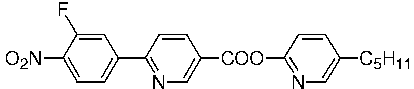 | 9.9 |
| 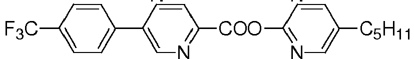 | 5 |
| 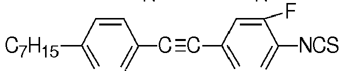 | 20 |
| 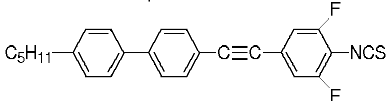 | 5 |
| Cetyltrimethylammonium perchlorate | 0.1 |

The mischcrystal was heated to be clear, filled in vacuum into a 2.8-inch screen, sealed and bonded. The contrast was tested to be 18:1, no residual image occurred after aging 5000 times, and the drive voltage was 30 to 40 V.
Embodiment 11 Formulation and performance of a smectic A phase liquid crystals obtained by introducing a low viscosity liquid crystal material for lowering the drive voltage into a formulation with a heterocyclic ring as the main body and added with any ester liquid crystal

**Table 12 Composition of the mischcrystal of Embodiment 11**

| Materials | Content, wt% |
|---|---|
| 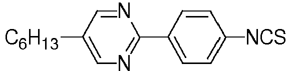 | 30 |
| 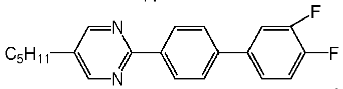 | 5 |
| 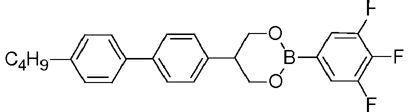 | 10 |
| 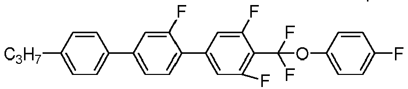 | 15 |
| 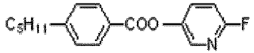 | 9.9 |
| 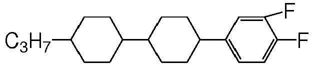 | 25 |
| 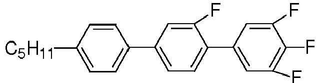 | 5 |
| Cetyltrimethylammonium perchlorate | 0.1 |

The mischcrystal was heated to be clear, filled in vacuum into a 2.8-inch screen, sealed and bonded. The contrast was tested to be 11:1, no residual image occurred after aging 5000 times, and the drive voltage was 20 to 30 V.
Embodiment 12 Formulation and performance of a smectic A phase liquid crystal obtained by introducing a dichroic dye for color display into a formulation with a heterocyclic ring as the main body and added with any ester liquid crystal

**Table 13 Composition of the mischcrystal of Embodiment 12 (comparative)**

| Materials | Content, wt% |
|---|---|
| 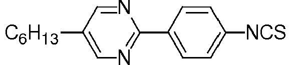 | 30 |
| 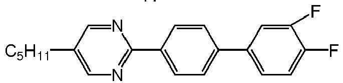 | 5 |
| 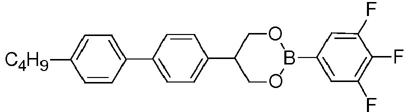 | 10 |
| 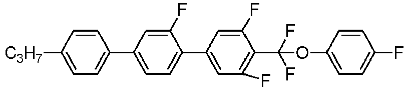 | 15 |
| 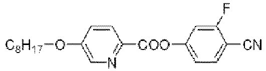 | 9.9 |
| 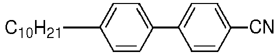 | 27 |
| 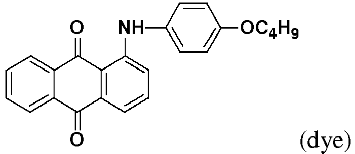 | 3 |
| Cetyltrimethylammonium perchlorate | 0.1 |

The mischcrystal was heated to be clear, filled in vacuum into a 2.8-inch screen, sealed and bonded to give a purple-red display screen. The contrast was tested to be 12:1, no residual image occurred after aging 5000 times, and the drive voltage was 30 to 40 V.
Embodiment 13 Formulation and performance of a smectic A phase liquid crystal obtained by introducing a UV glue for reducing the working temperature and enhancing the adhesion of the liquid crystal cell into a formulation with a heterocyclic ring as the main body and added with any ester liquid crystal

**Table 14 Composition of the mischcrystal of Embodiment 13 (comparative)**

| Materials | Content, wt% |
|---|---|
| 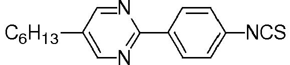 | 30 |
| 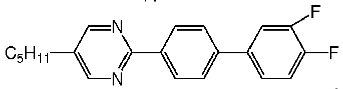 | 5 |
| 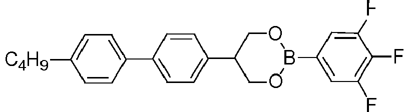 | 10 |
| 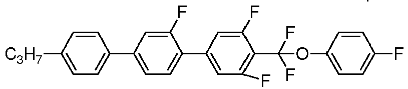 | 15 |
| 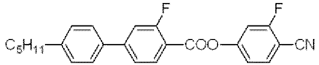 | 9.9 |
| 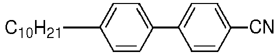 | 20 |
| NoA65 (UV glue) | 10 |
| Cetyltrimethylammonium perchlorate | 0.1 |

The mischcrystal was in a clearing state at room temperature, was filled in vacuum into the 2.8-inch screen at room temperature, sealed and bonded, solidified by UV to give a smectic A phase liquid crystal display screen. The contrast was tested to be 11:1, no residual image occurred after aging 5000 times, and the drive voltage was 30 to 40 V.
Embodiment 14 Formulation and performance of a smectic A phase liquid crystal with any combination of heterocyclic rings as the main body and without any ester liquid crystal added

**Table 15 Composition of the mischcrystal of Embodiment 14 (comparative)**

| Materials | Content, wt% |
|---|---|
| 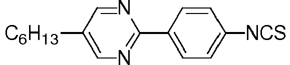 | 30 |
| 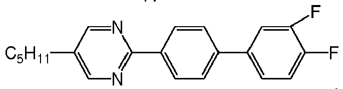 | 5 |
| 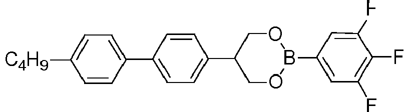 | 10 |
| 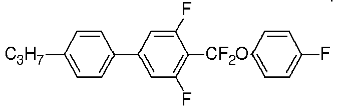 | 15 |
| 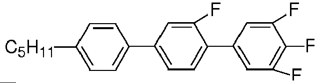 | 19.9 |
| 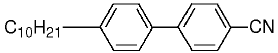 | 20 |
| Cetyltrimethylammonium perchlorate | 0.1 |

The mischcrystal was heated to be clear, filled in vacuum into a 2.8-inch screen, sealed and bonded. The contrast was tested to be 11:1, no residual image occurred after aging 5000 times, and the drive voltage was 40 to 50 V.
Embodiment 15 Formulation and performance of a smectic A phase liquid crystal with a nitrogen-containing heterocyclic ring as the main body and without any ester liquid crystal added

**Table 16 Composition of the mischcrystal of Embodiment 15 (comparative)**

| Materials | Content, wt% |
|---|---|
| 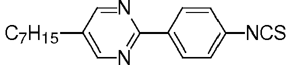 | 15 |
| 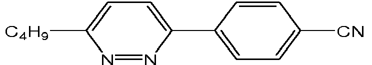 | 25 |
| 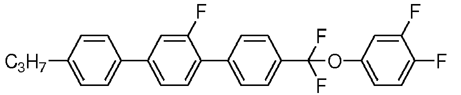 | 20 |
| 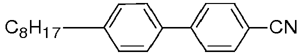 | 9.9 |
| 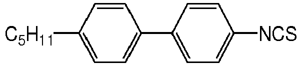 | 25 |
| 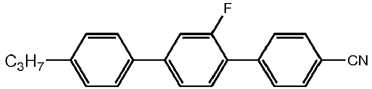 | 5 |
| Cetyldimethylbenzylammonium chloride | 0.1 |

The mischcrystal was heated to be clear, filled in vacuum into a 2.8-inch screen, sealed and bonded. The contrast was tested to be 11:1, no residual image occurred after aging 5000 times, and the drive voltage was 40 to 50 V.
Embodiment 16 Formulation and performance of a smectic A phase liquid crystal with a pyridine heterocyclic ring as the main body and without any ester liquid crystal added

**Table 17 Composition of the mischcrystal of Embodiment 16 (comparative)**

| Materials | Content, wt% |
|---|---|
| 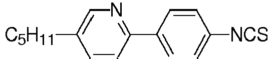 | 40 |
| 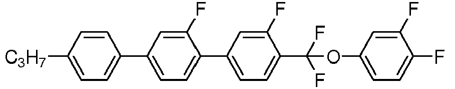 | 20 |
| 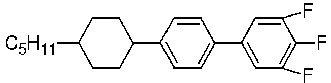 | 9.9 |
| 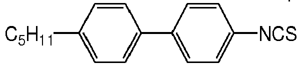 | 25 |
| 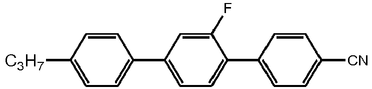 | 5 |
| Cetyltrimethylammonium bromide | 0.1 |

The mischcrystal was heated to be clear, filled in vacuum into a 2.8-inch screen, sealed and bonded. The contrast was tested to be 11:1, no residual image occurred after aging 5000 times, and the drive voltage was 40 to 50 V.
Embodiment 17 Formulation and performance of a smectic A phase liquid crystal with a pyrimidine heterocyclic ring as the main body and without any ester liquid crystal added

**Table 18 Composition of the mischcrystal of Embodiment 17 (comparative)**

| Materials | Content, wt% |
|---|---|
| 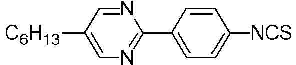 | 40 |
| 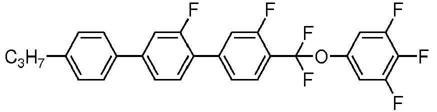 | 20 |
| 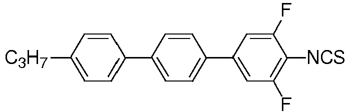 | 9.9 |
| 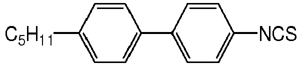 | 25 |
| 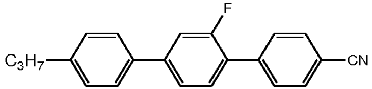 | 5 |
| Cetyltributylammonium bromide | 0.1 |

The mischcrystal was heated to be clear, filled in vacuum into a 2.8-inch screen, sealed and bonded. The contrast was tested to be 11:1, no residual image occurred after aging 5000 times, and the drive voltage was 40 to 50 V.
Embodiment 18 Formulation and performance of a smectic A phase liquid crystal with a oxygen-containing heterocyclic ring as the main body and without any ester liquid crystal added

**Table 19: Composition of the mischcrystal of Embodiment 18 (comparative)**

| Materials | Content, wt% |
|---|---|
| 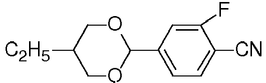 | 15 |
| 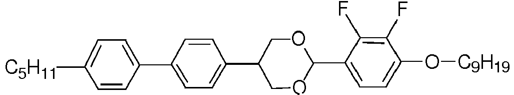 | 25 |
| 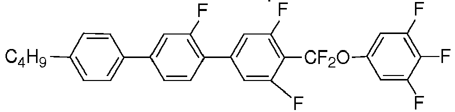 | 20 |
| 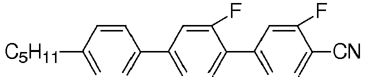 | 9.9 |
| 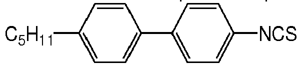 | 25 |
| 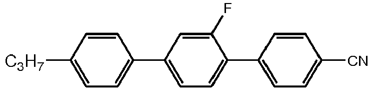 | 5 |
| Dodecyl pyridine bromide | 0.1 |

The mischcrystal was heated to be clear, filled in vacuum into a 2.8-inch screen, sealed and bonded. The contrast was tested to be 11:1, no residual image occurred after aging 5000 times, and the drive voltage was 40 to 50 V.
Embodiment 19 Formulation and performance of a smectic A phase liquid crystal with a sulfur-containing heterocyclic ring as the main body and without any of ester liquid crystals, and its performance

**Table 20 Composition of the mischcrystal of Embodiment 19 (comparative)**

| Materials | Content, wt% |
|---|---|
| 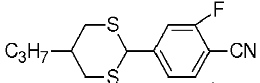 | 15 |
| 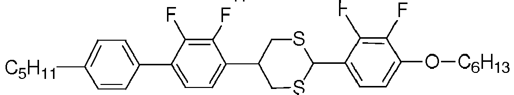 | 25 |
| 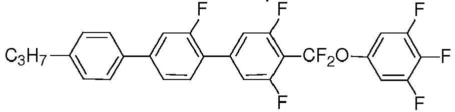 | 20 |
| 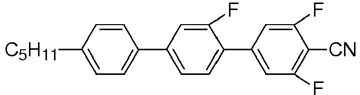 | 9.9 |
| 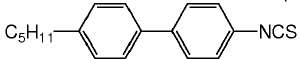 | 25 |
| 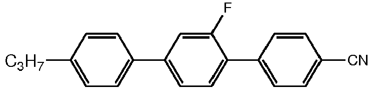 | 5 |
| Cetyl pyridine bromide | 0.1 |

The mischcrystal was heated to be clear, filled in vacuum into a 2.8-inch screen, sealed and bonded. The contrast was tested to be 11:1, no residual image occurred after aging 5000 times, and the drive voltage was 40 to 50 V.
Embodiment 20 Formulation and performance of a smectic A phase liquid crystal with a boron-containing heterocyclic ring as the main body and without any ester liquid crystal added

**Table 21 Composition of the mischcrystal of Embodiment 20 (comparative)**

| Materials | Content, wt% |
|---|---|
| 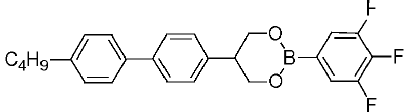 | 15 |
| 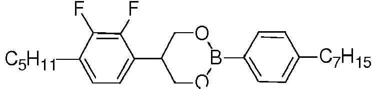 | 25 |
| 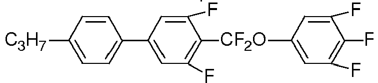 | 20 |
| 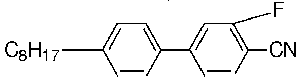 | 14.9 |
| 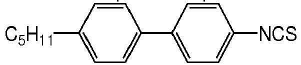 | 20 |
| 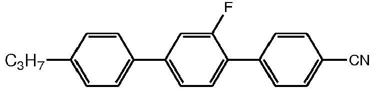 | 5 |
| Cetyl pyridine chloride | 0.1 |

The mischcrystal was heated to be clear, filled in vacuum into a 2.8-inch screen, sealed and bonded. The contrast was tested to be 11:1, no residual image occurred after aging 5000 times, and the drive voltage was 40 to 50 V.

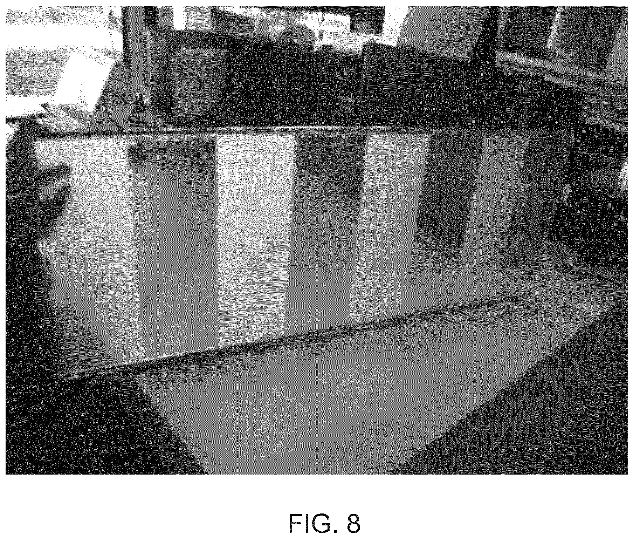

The smectic A phase liquid crystal formulation of the present invention also may be used as a dimming media, and a dimming glass having a large size can be obtained by increasing the pixel and increasing the size of the glass (as shown in FIG. 8).

The new generation smectic A phase liquid crystal material formulation of the present invention is different from the first generation smectic A phase liquid crystal and the second generation smectic A phase liquid crystal in the working principle, and are significantly superior to the previous smectic A phase formulations in practical effects. By applying the smectic A phase formulation of the present invention to a display device, the displayed image can be completely erased without any residual images, and the image can be completely erased after long-term drive and long-term use, the contrast is high and is generally higher than 10, and the drive voltage is low and is generally 20 to 50 V.

## Claims

1. A smectic A phase liquid crystal material, comprising at least one heterocyclic compound of Formula (III), (IV), (V), (VI) or (VII): 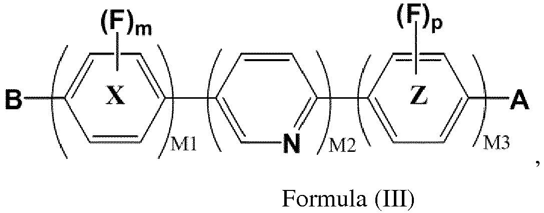 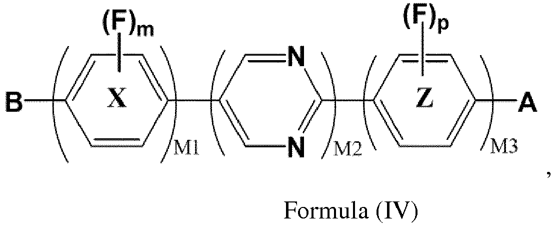 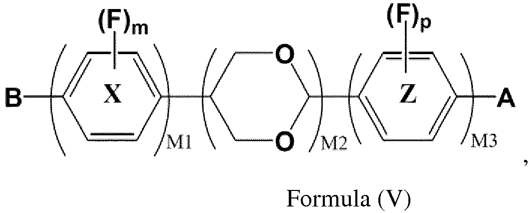 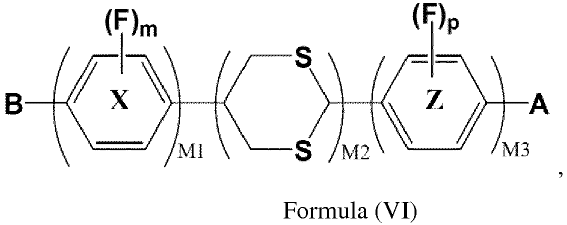 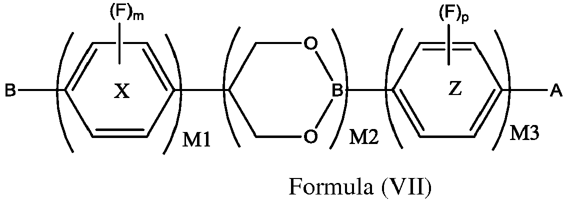
wherein for formulas (III)-(VII), m and p are independently 0, 1, 2, 3, or 4, M2 is 1 or 2, and M3 is 1 or 2;
X and Z are substituted or unsubstituted phenyl rings,
F is a fluorine atom, substituting any one or more of hydrogen atoms on the phenyl ring of X or Z; and
for formulas (III)-(IV) and (VI)-(VII), M1 is 0, 1 or 2; and
for formula (V), M1 is 1 or 2; and
for formula (III), B is selected from the group consisting of: CN, F, NCS, NCO, CF₃, CHF₂, CH₂F, OCF₃, OCHF₂, OCH₂F, NO₂, Cl, CH=CF₂ and OCH=CF₂; C1-C20 alkyl, C2-C20 alkenyloxy, C1-C20 silanyl and C1-C20 siloxanyl, and the halogenated groups thereof; and A is selected from the group consisting of CN, F, NCS, NCO, CF₃, CHF₂, CH₂F, OCF₃, OCHF₂, OCH₂F, NO₂, Cl, CH=CF₂ and OCH=CF₂; C1-C20 alkyl, C2-C20 alkenyl, C2-C20 alkenyloxy, C1-C20 silanyl and C1-C20 siloxanyl, and the halogenated groups thereof;
for formula (IV), B is selected from the group consisting of: CN, F, NCS, NCO, CF₃, CHF₂, CH₂F, OCF₃, OCHF₂, OCH₂F, NO₂, Cl, CH=CF₂ and OCH=CF₂; C2-C20 alkenyloxy, C1-C20 silanyl and C1-C20 siloxanyl, and the halogenated groups thereof; and A is selected from the group consisting of: F, NCS, NCO, CF₃, CHF₂, CH₂F, OCF₃, OCHF₂, OCH₂F, NO₂, Cl, CH=CF₂ and OCH=CF₂; C2-C20 alkenyl, C2-C20 alkenyloxy, C1-C20 silanyl and C1-C20 siloxanyl, and the halogenated groups thereof; and
for formula (V), B is selected from the group consisting of: CN, F, NCS, NCO, CF₃, CHF₂, CH₂F, OCF₃, OCHF₂, OCH₂F, NO₂, Cl, CH=CF₂ and OCH=CF₂; C1-C20 alkyl, C1-C20 alkoxy, C2-C20 alkenyloxy, C1-C20 silanyl and C1-C20 siloxanyl, and the halogenated groups thereof; and A is selected from the group consisting of: CN, F, NCO, CF₃, CHF₂, CH₂F, OCF₃, OCHF₂, OCH₂F, NO₂, Cl, CH=CF₂ and OCH=CF₂; C1-C20 alkyl, C2-C20 alkenyl, C2-C20 alkenyloxy, C1-C20 silanyl and C1-C20 siloxanyl, and the halogenated groups thereof; and
for formula (VI), B is selected from the group consisting of: CN, F, NCS, NCO, CF₃, CHF₂, CH₂F, OCF₃, OCHF₂, OCH₂F, NO₂, Cl, CH=CF₂ and OCH=CF₂; C1-C20 alkyl, C2-C20 alkenyloxy, C1-C20 silanyl and C1-C20 siloxanyl, and the halogenated groups thereof; and A is selected from the group consisting of: F, NCS, NCO, CF₃, CHF₂, CH₂F, OCF₃, OCHF₂, OCH₂F, NO₂, Cl, CH=CF₂ and OCH=CF₂; C1-C20 alkyl, C2-C20 alkenyl, C2-C20 alkenyloxy, C1-C20 silanyl and C1-C20 siloxanyl, and the halogenated groups thereof; and
for formula (VII), B is selected from the group consisting of: CN, F, NCS, NCO, CF₃, CHF₂, CH₂F, OCF₃, OCHF₂, OCH₂F, NO₂, Cl, CH=CF₂ and OCH=CF₂; C1-C20 alkyl, C1-C20 alkoxy, C2-C20 alkeny, C2-C20 alkenyloxy, C1-C20 silanyl and C1-C20 siloxanyl, and the halogenated groups thereof; and A is selected from the group consisting of: F, NCS, NCO, CF₃, CHF₂, CH₂F, OCF₃, OCHF₂, OCH₂F, NO₂, Cl and OCH=CF₂; C2-C20 alkenyl, C2-C20 alkenyloxy, C1-C20 silanyl and C1-C20 siloxanyl;
and wherein the smectic A phase liquid crystal material further comprises at least one ester compound of Formula (II): 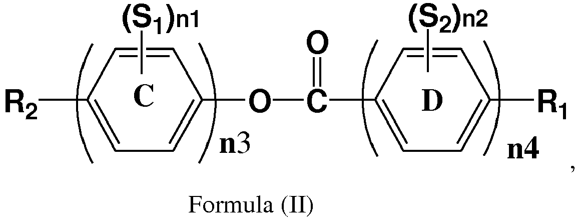
wherein n₁ and n₂ are independently 0, 1, 2, 3, or 4,
n₃ is 0, 1, or 2, and n₄ is 0, 1, 2, or 3;
C and D are independently substituted or unsubstituted phenyl rings;
S₁ and S₂ are independently a N atom or a F atom, replacing any one or more of C atoms on the phenyl ring when S₁ or S₂ is a N atom, and replacing any one or more of H atoms on the phenyl ring when S₁ or S₂ is a F atom;
R₁ and R₂ are independently selected from the group consisting of: F, NCS, NCO, CF₃, CHF₂, CH₂F, OCF₃, OCHF₂, OCH₂F, NO₂, Cl, CH=CF₂ and OCH=CF₂; C1-C20 alkyl, C1-C20 alkoxy, C1-C20 alkenyl, C1-C20 alkenyloxy, C1-C20 silanyl and C1-C20 siloxanyl, and the halogenated groups thereof; C1-C20 alkyl, C1-C20 alkoxy, C1-C20 alkenyl, C1-C20 alkenyloxy and isomers thereof with any -CH₂- substituted with -O-, -S-, -CF₂-, -CF₂O-, -CO-, -O-COO-, -CF=CF-, -CH=CF-, - CF=CH- and -CH=CH-.

2. The smectic A phase liquid crystal material of claim 1, further comprising at least one ionic compound.

3. The smectic A phase liquid crystal material of claim 2, further comprising nematic liquid crystal formation, nematic liquid crystal monomers, or rod-like compounds.

4. The smectic A phase liquid crystal material of claim 3, further comprising a dichroic dye or a UV glue.

5. The smectic A phase liquid crystal material of claim 1, wherein the ester compound of Formula (II) is at least one of the compounds of Formulas (VIII), (XIII) and (XIV), 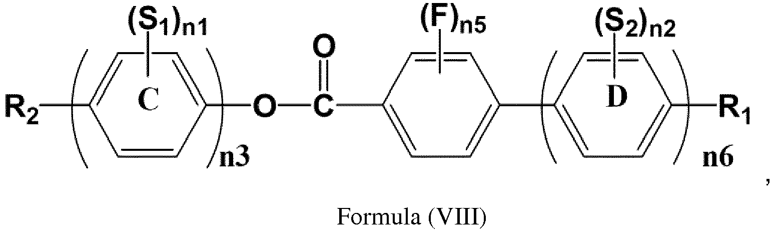 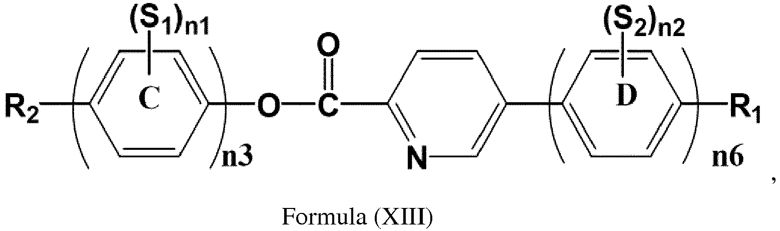 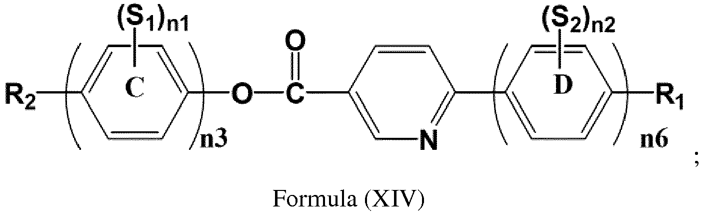 wherein n₅ is 0, 1, 2, 3, or 4, and n₆ is 0, 1, or 2.

6. The smectic A phase liquid crystal material of claim 2, wherein the ionic compound is selected from the group consisting of: sodium lauryl sulfate, ethyltriphenylphosphonium iodide, (ferrocenylmethyl)trimethylammonium iodide, 1,2-dimethyl-3-butylimidazole hexafluorophosphate, tetraethylamine para-toluenesulfonate, phenyltriethylammonium iodide, 1-octyl-3-methylimidazole hexafluorophosphate, bis(tetra-n-butylamine)bis(1,3-dithiole-2-thione-4,5-dithiol)palladium, tetra-n-butyl bis(1,3-dithiole-2-thione-4,5-dithiol)nickel, bis(tetra-n-butylammonium) bis(1,3-dithiole-2-thione-4,5-dithiol)zinc, bis(tetra-n-butylammonium)tetracyanodiphenoquinodimethane, tetrabutylammonium bromide, cetylammonium perchlorate, cetyltetraammonium bromide, 1-butyl-3-methylimidazole tetrachloride ferrate, methyltriphenylphosphonium iodide, tetraphenylphosphonium iodide, cetyltrimethyl ammonium bromide, cetyltrimethyl ammonium perchlorate, cetyldimethylbenzylammonium chloride, dodecyl pyridine bromide, cetyl pyridine bromide, cetyl pyridine chloride, and cetyltributylammonium bromide.

7. The smectic A phase liquid crystal material of claim 6, further comprising an ionic compound selected from the group consisting of: sodium lauryl sulfate, ethyltriphenylphosphonium iodide, (ferrocenylmethyl)trimethylammonium iodide, 1,2-dimethyl-3-butylimidazole hexafluorophosphate, tetraethylamine para-toluenesulfonate, phenyltriethylammonium iodide, 1-octyl-3-methylimidazole hexafluorophosphate, bis(tetra-n-butylamine)bis(1,3-dithiole-2-thione-4,5-dithiol)palladium, tetra-n-butyl bis(1,3-dithiole-2-thione-4,5-dithiol)nickel, bis(tetra-n-butylammonium)bis(1,3-dithiole-2-thione-4,5-dithiol)zinc, bis(tetra-n-butylammonium)tetracyanodiphenoquinodimethane, tetrabutylammonium bromide, cetylammonium perchlorate, cetyltetraammonium bromide, 1-butyl-3-methylimidazole tetrachloride ferrate, methyltriphenylphosphonium iodide, tetraphenylphosphonium iodide, cetyltrimethyl ammonium bromide, cetyltrimethyl ammonium perchlorate, cetyldimethylbenzylammonium chloride, dodecyl pyridine bromide, cetyl pyridine bromide, cetyl pyridine chloride, and cetyltributylammonium bromide.

8. A device comprising a smectic A phase liquid crystal material, wherein the smectic A phase liquid crystal material comprises at least one heterocyclic compound of Formula (III), (IV), (V), (VI) or (VII): 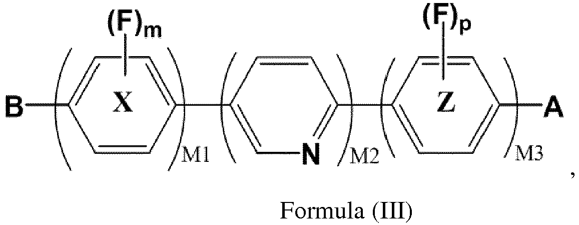 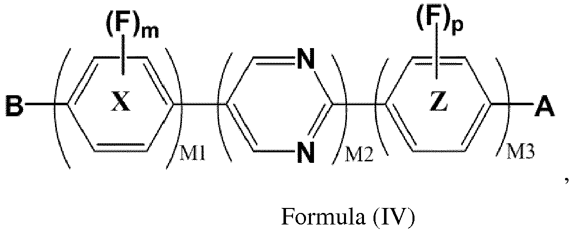 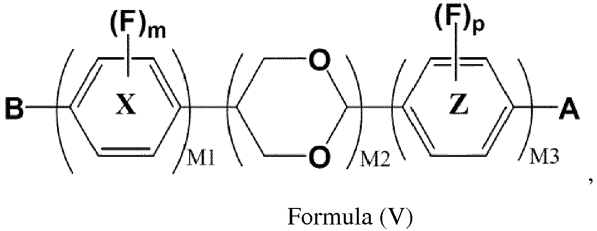 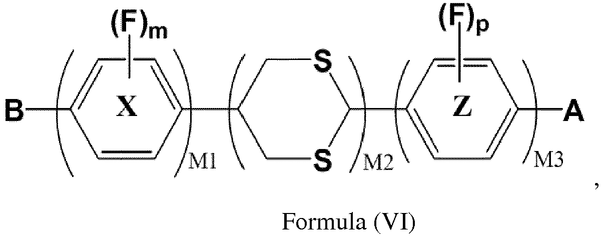 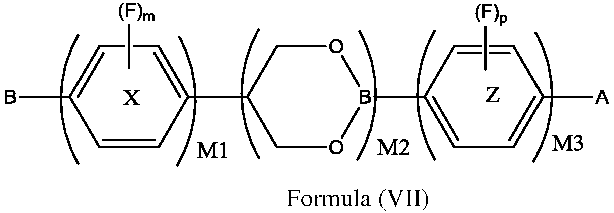
wherein for formulas (III)-(VII), m and p are independently 0, 1, 2, 3, or 4, M2 is 1 or 2, and M3 is 1 or 2;
X and Z are substituted or unsubstituted phenyl rings,
F is a fluorine atom, substituting any one or more of hydrogen atoms on the phenyl ring of X or Z; and
for formulas (III)-(IV) and (VI)-(VII), M1 is 0, 1 or 2; and
for formula (V), M1 is 1 or 2; and
for formula (III), B is selected from the group consisting of: CN, F, NCS, NCO, CF₃, CHF₂, CH₂F, OCF₃, OCHF₂, OCH₂F, NO₂, Cl, CH=CF₂ and OCH=CF₂; C1-C20 alkyl, C2-C20 alkenyloxy, C1-C20 silanyl and C1-C20 siloxanyl, and the halogenated groups thereof; and A is selected from the groups consisting of CN, F, NCS, NCO, CF₃, CHF₂, CH₂F, OCF₃, OCHF₂, OCH₂F, NO₂, Cl, CH=CF₂ and OCH=CF₂; C1-C20 alkyl, C2-C20 alkenyl, C2-C20 alkenyloxy, C1-C20 silanyl and C1-C20 siloxanyl, and the halogenated groups thereof;
for formula (IV), B is selected from the group consisting of: CN, F, NCS, NCO, CF₃, CHF₂, CH₂F, OCF₃, OCHF₂, OCH₂F, NO₂, Cl, CH=CF₂ and OCH=CF₂; C2-C20 alkenyloxy, C1-C20 silanyl and C1-C20 siloxanyl, and the halogenated groups thereof; and A is selected from the group consisting of: F, NCS, NCO, CF₃, CHF₂, CH₂F, OCF₃, OCHF₂, OCH₂F, NO₂, Cl, CH=CF₂ and OCH=CF₂; C2-C20 alkenyl, C2-C20 alkenyloxy, C1-C20 silanyl and C1-C20 siloxanyl, and the halogenated groups thereof; and
for formula (V), B is selected from the group consisting of: CN, F, NCS, NCO, CF₃, CHF₂, CH₂F, OCF₃, OCHF₂, OCH₂F, NO₂, Cl, CH=CF₂ and OCH=CF₂; C1-C20 alkyl, C1-C20 alkoxy, C2-C20 alkenyloxy, C1-C20 silanyl and C1-C20 siloxanyl, and the halogenated groups thereof; and A is selected from the group consisting of: CN, F, NCO, CF₃, CHF₂, CH₂F, OCF₃, OCHF₂, OCH₂F, NO₂, Cl, CH=CF₂ and OCH=CF₂; C1-C20 alkyl, C2-C20 alkenyl, C2-C20 alkenyloxy, C1-C20 silanyl and C1-C20 siloxanyl, and the halogenated groups thereof; and
for formula (VI), B is selected from the group consisting of: CN, F, NCS, NCO, CF₃, CHF₂, CH₂F, OCF₃, OCHF₂, OCH₂F, NO₂, Cl, CH=CF₂ and OCH=CF₂; C1-C20 alkyl, C2-C20 alkenyloxy, C1-C20 silanyl and C1-C20 siloxanyl, and the halogenated groups thereof; and A is selected from the group consisting of: F, NCS, NCO, CF₃, CHF₂, CH₂F, OCF₃, OCHF₂, OCH₂F, NO₂, Cl, CH=CF₂ and OCH=CF₂; C1-C20 alkyl, C2-C20 alkenyl, C2-C20 alkenyloxy, C1-C20 silanyl and C1-C20 siloxanyl, and the halogenated groups thereof; and
for formula (VII), B is selected from the group consisting of: CN, F, NCS, NCO, CF₃, CHF₂, CH₂F, OCF₃, OCHF₂, OCH₂F, NO₂, Cl, CH=CF₂ and OCH=CF₂; C1-C20 alkyl, C1-C20 alkoxy, C2-C20 alkeny, C2-C20 alkenyloxy, C1-C20 silanyl and C1-C20 siloxanyl, and the halogenated groups thereof; and A is selected from the group consisting of: F, NCS, NCO, CF₃, CHF₂, CH₂F, OCF₃, OCHF₂, OCH₂F, NO₂, Cl and OCH=CF₂; C2-C20 alkenyl, C2-C20 alkenyloxy, C1-C20 silanyl and C1-C20 siloxanyl,
and wherein the device has a capacitor capable of applying electric energy in dual waveforms, one waveform is high-frequency drive pulses for clearing operation, and the other waveform is low-frequency drive pulses for frosting operation, and
wherein the smectic A phase liquid crystal material further comprises at least one ester compound of Formula (II): 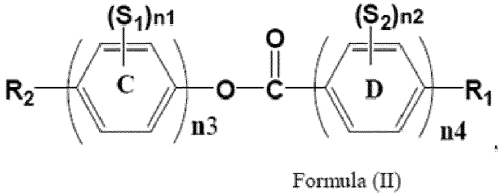 wherein
n₁ and n₂ are independently 0, 1, 2, 3, or 4,
n₃ is 0, 1, or 2, and n₄ is 0, 1, 2, or 3;
C and D are independently substituted or unsubstituted phenyl rings;
S₁ and S₂ are independently a N atom or a F atom, replacing any one or more of C atoms on the phenyl ring when S₁ or S₂ is a N atom, and replacing any one or more of H atoms on the phenyl ring when S₁ or S₂ is a F atom; and
R₁ and R₂ are independently selected from the group consisting of: F, NCS, NCO, CF₃, CHF₂, CH₂F, OCF₃, OCHF₂, OCH₂F, NO₂, Cl, CH=CF₂ and OCH=CF₂; C1-C20 alkyl, C1-C20 alkoxy, C1-C20 alkenyl, C1-C20 alkenyloxy, C1-C20 silanyl and C1-C20 siloxanyl, and the halogenated groups thereof; C1-C20 alkyl, C1-C20 alkoxy, C1-C20 alkenyl, C1-C20 alkenyloxy and isomers thereof with any -CH₂- substituted with -O-, -S-, - CF₂-, -CF₂O-, -CO-, -O-COO-, -CF=CF-, -CH=CF-, - CF=CH- and -CH=CH-.

9. The device of claim 8, wherein the ester compound of Formula (II) is at least one of the compounds of Formulas (VIII), (XIII) and (XIV), 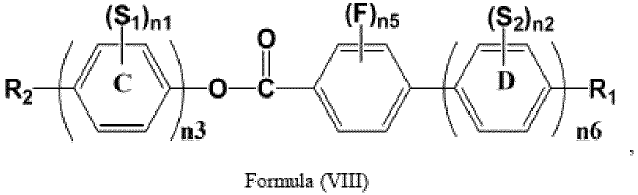 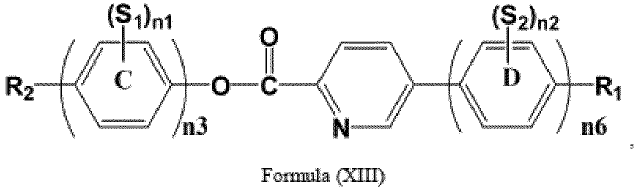 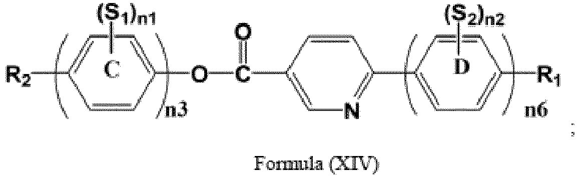 wherein n₅ is 0, 1, 2, 3, or 4, and n₆ is 0, 1, or 2.

## Patentansprüche

1. Smektisches A-Phasen-Flüssigkristallmaterial, das mindestens eine heterozyklische Verbindung der Formel (III), (IV), (V), (VI) oder (VII) umfasst: 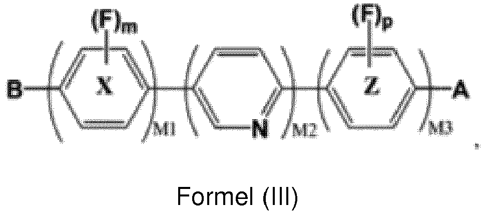 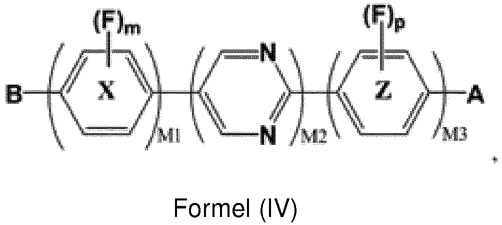 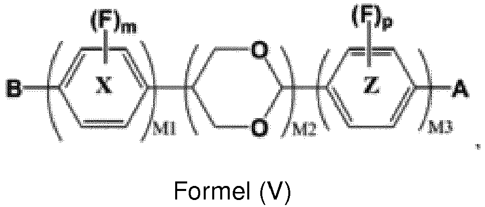 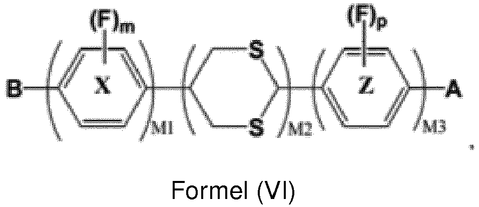 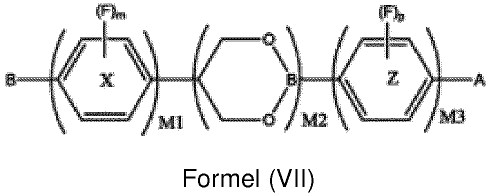
wobei für die Formeln (III)-(VII) m und p unabhängig voneinander 0, 1, 2, 3 oder 4 sind, M2 1 oder 2 ist und M3 1 oder 2 ist;
X und Z substituierte oder unsubstituierte Phenylringe sind,
F ein Fluoratom ist, das eines oder mehrere der Wasserstoffatome am Phenylring von X oder Z substituiert; und
für die Formeln (III)-(IV) und (VI)-(VII) M1 0, 1 oder 2 ist; und
für Formel (V) M1 1 oder 2 ist; und
für Formel (III) B ausgewählt ist aus der Gruppe, bestehend aus: CN, F, NCS, NCO, CF₃, CHF₂, CH₂F, OCF₃, OCHF₂, OCH₂F, NO₂, Cl, CH=CF₂ und OCH=CF₂; C1-C20 Alkyl, C2-C20 Alkenyloxy, C1-C20 Silanyl und C1-C20 Siloxanyl sowie deren halogenierten Gruppen; und A ausgewählt ist aus der Gruppe, bestehend aus CN, F, NCS, NCO, CF₃, CHF₂, CH₂F, OCF₃, OCHF₂, OCH₂F, NO₂, Cl, CH=CF₂ und OCH=CF₂; C1-C20 Alkyl, C2-C20 Alkenyl, C2-C20 Alkenyloxy, C1-C20 Silanyl und C1-C20 Siloxanyl sowie deren halogenierten Gruppen;
für Formel (IV) B ausgewählt ist aus der Gruppe, bestehend aus: CN, F, NCS, NCO, CF₃, CHF₂, CH₂F, OCF₃, OCHF₂, OCH₂F, NO₂, Cl, CH=CF₂ und OCH=CF₂; C2-C20 Alkenyloxy, C1-C20 Silanyl und C1-C20 Siloxanyl und deren halogenierten Gruppen; und A ausgewählt ist aus der Gruppe, bestehend aus: F, NCS, NCO, CF₃, CHF₂, CH₂F, OCF₃, OCHF₂, OCH₂F, NO₂, Cl, CH=CF₂ und OCH=CF₂; C2-C20 Alkenyl, C2-C20 Alkenyloxy, C1-C20 Silanyl und C1-C20 Siloxanyl und deren halogenierten Gruppen; und
für Formel (V) B ausgewählt ist aus der Gruppe, bestehend aus: CN, F, NCS, NCO, CF₃, CHF₂, CH₂F, OCF₃, OCHF₂, OCH₂F, NO₂, Cl, CH=CF₂ und OCH=CF₂; C1-C20 Alkyl, C1-C20 Alkoxy, C2-C20 Alkenyloxy, C1-C20 Silanyl und C1-C20 Siloxanyl sowie deren halogenierten Gruppen; und A ausgewählt ist aus der Gruppe, bestehend aus: CN, F, NCO, CF₃, CHF₂, CH₂F, OCF₃, OCHF₂, OCH₂F, NO₂, Cl, CH=CF₂ und OCH=CF₂; C1-C20 Alkyl, C2-C20 Alkenyl, C2-C20 Alkenyloxy, C1-C20 Silanyl und C1-C20 Siloxanyl sowie deren halogenierten Gruppen; und
für Formel (VI) B ausgewählt ist aus der Gruppe, bestehend aus: CN, F, NCS, NCO, CF₃, CHF₂, CH₂F, OCF₃, OCHF₂, OCH₂F, NO₂, Cl, CH=CF₂ und OCH=CF₂; C1-C20 Alkyl, C2-C20 Alkenyloxy, C1-C20 Silanyl und C1-C20 Siloxanyl sowie deren halogenierten Gruppen; und A ausgewählt ist aus der Gruppe, bestehend aus: F, NCS, NCO, CF₃, CHF₂, CH₂F, OCF₃, OCHF₂, OCH₂F, NO₂, Cl, CH=CF₂ und OCH=CF₂; C1-C20 Alkyl, C2-C20 Alkenyl, C2-C20 Alkenyloxy, C1-C20 Silanyl und C1-C20 Siloxanyl sowie deren halogenierten Gruppen; und
für Formel (VII) B ausgewählt ist aus der Gruppe, bestehend aus: CN, F, NCS, NCO, CF₃, CHF₂, CH₂F, OCF₃, OCHF₂, OCH₂F, NO₂, Cl, CH=CF₂ und OCH=CF₂; C1-C20 Alkyl, C1-C20 Alkoxy, C2-C20 Alkenyl, C2-C20 Alkenyloxy, C1-C20 Silanyl und C1-C20 Siloxanyl sowie deren halogenierten Gruppen; und A ausgewählt ist aus der Gruppe, bestehend aus: F, NCS, NCO, CF₃, CHF₂, CH₂F, OCF₃, OCHF₂, OCH₂F, NO₂, Cl und OCH=CF₂; C2-C20 Alkenyl, C2-C20 Alkenyloxy, C1-C20 Silanyl und C1-C20 Siloxanyl;
und wobei das smektische A-Phasen-Flüssigkristallmaterial ferner mindestens eine Esterverbindung der Formel (II) umfasst: 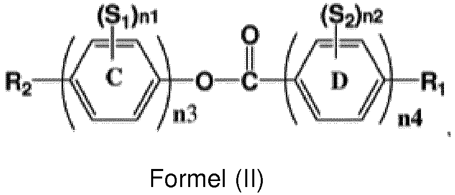
worin n₁ und n₂ unabhängig voneinander 0, 1, 2, 3 oder 4 sind,
n₃ 0, 1 oder 2 ist und n₄ 0, 1, 2 oder 3 ist;
C und D unabhängig voneinander substituierte oder unsubstituierte Phenylringe sind;
S₁ und S₂ unabhängig voneinander ein N-Atom oder ein F-Atom sind, wobei eines oder mehrere der C-Atome am Phenylring ersetzt werden, wenn S₁ oder S₂ ein N-Atom ist, und eines oder mehrere der H-Atome am Phenylring ersetzt werden, wenn S₁ oder S₂ ein F-Atom ist;
R₁ und R₂ unabhängig voneinander ausgewählt sind aus der Gruppe, bestehend aus: F, NCS, NCO, CF₃, CHF₂, CH₂F, OCF₃, OCHF₂, OCH₂F, NO₂, Cl, CH=CF₂ und OCH=CF₂; C1-C20 Alkyl, C1-C20 Alkoxy, C1-C20 Alkenyl, C1-C20 Alkenyloxy, C1-C20 Silanyl und C1-C20 Siloxanyl sowie deren halogenierten Gruppen; C1-C20 Alkyl, C1-C20 Alkoxy, C1-C20-Alkenyl, C1-C20 Alkenyloxy und Isomeren davon, wobei ein beliebiges -CH₂-, substituiert ist mit -O-, -S-, -CF₂, -CF₂O-, - CO-, -O-COO-, -CF=CF-, -CH=CF-, -CF=CH- und -CH=CH-.

2. Smektisches A-Phasen-Flüssigkristallmaterial nach Anspruch 1, das ferner mindestens eine ionische Verbindung umfasst.

3. Smektisches A-Phasen-Flüssigkristallmaterial nach Anspruch 2, das ferner eine nematische Flüssigkristallformation, nematische Flüssigkristallmonomere oder stabartige Verbindungen umfasst.

4. Smektisches A-Phasen-Flüssigkristallmaterial nach Anspruch 3, das ferner einen dichroitischen Farbstoff oder einen UV-Klebstoff umfasst.

5. Smektisches A-Phasen-Flüssigkristallmaterial nach Anspruch 1, wobei die Esterverbindung der Formel (II) mindestens eine der Verbindungen der Formeln (VIII), (XIII) und (XIV) ist, 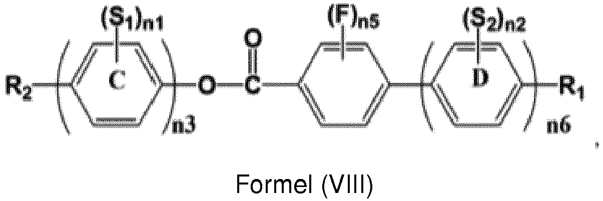 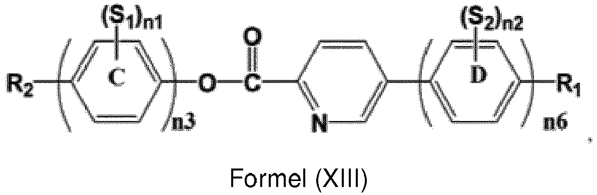 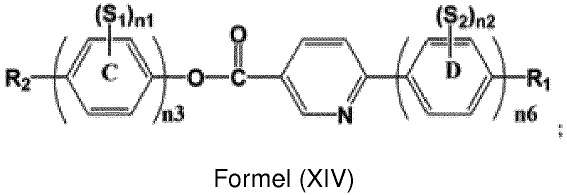 worin n₅ 0, 1, 2, 3 oder 4 ist und n₆ 0, 1 oder 2 ist.

6. Smektisches A-Phasen-Flüssigkristallmaterial nach Anspruch 2, wobei die ionische Verbindung ausgewählt ist aus der Gruppe bestehend aus: Natriumlaurylsulfat, Ethyltriphenylphosphoniumjodid, (Ferrocenylmethyl)trimethylammoniumjodid, 1,2-Dimethyl-3-butylimidazol-hexafluorphosphat, Tetraethylamin-paratoluolsulfonat, Phenyltriethylammoniumiodid, 1-Octyl-3-methylimidazol-hexafluorphosphat, Bis(tetra-n-butylamin)bis(1,3-dithiol-2-thion-4,5-dithiol)palladium, Tetra-n-butyl-bis(1,3-dithiol-2-thion-4,5-dithiol)nickel, Bis(tetra-n-butylammonium)bis(1,3-dithiol-2-thion-4,5-dithiol)zink, Bis(tetra-n-butylammonium)tetracyano-diphenoquino-dimethan, Tetrabutylammoniumbromid, Cetylammoniumperchlorat, Cetyltetrammoniumbromid, 1-Butyl-3-methylimidazol-tetrachloridferrat, Methyltriphenylphosphoniumjodid, Tetraphenylphosphoniumjodid, Cetyltrimethylammoniumbromid, Cetyltrimethylammoniumperchlorat, Cetyldimethylbenzylammoniumchlorid, Dodecylpyridinbromid, Cetylpyridinbromid, Cetylpyridinchlorid und Cetyltributylammoniumbromid.

7. Smektisches A-Phasen-Flüssigkristallmaterial nach 6, das ferner eine ionische Verbindung umfasst, die ausgewählt ist aus der Gruppe, bestehend aus: Natriumlaurylsulfat, Ethyltriphenylphosphoniumjodid, (Ferrocenylmethyl)trimethylammoniumjodid, 1,2-Dimethyl-3-butylimidazol-hexafluorphosphat, Tetraethylamin-para-toluolsulfonat, Phenyltriethylammoniumjodid, 1-Octyl-3-methylimidazol-hexafluorphosphat, Bis(tetra-n-butylamin)bis(1,3-dithiol-2-thion-4,5-dithiol)palladium, Tetra-n-butyl-bis(1,3-dithiol-2-thion-4,5-dithiol)nickel, Bis(tetra-n-butylammonium)bis(1,3-dithiol-2-thion)-4,5-dithiol)zink, Bis(tetra-n-butylammonium)tetracyano-diphenochino-dimethan, Tetrabutylammoniumbromid, Cetylammoniumperchlorat, Cetyltetraammoniumbromid, 1-Butyl-3-methylimidazol-tetrachloridferrat, Methyltriphenylphosphoniumjodid, Tetraphenylphosphoniumjodid, Cetytrimethylammoniumbromid, Cetytrimethylammoniumperchlorat, Cetyldimethylbenzylammoniumchlorid, Dodecylpyridinbromid, Cetylpyridinbromid, Cetylpyridinchlorid und Cetyltributylammoniumbromid.

8. Vorrichtung, die ein smektisches A-Phasen-Flüssigkristallmaterial umfasst, wobei das smektische A-Phasen-Flüssigkristallmaterial mindestens eine heterozyklische Verbindung der Formel (III), (IV), (V), (VI) oder (VII) umfasst: 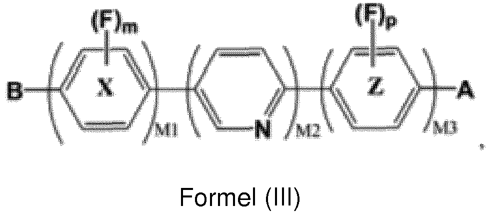 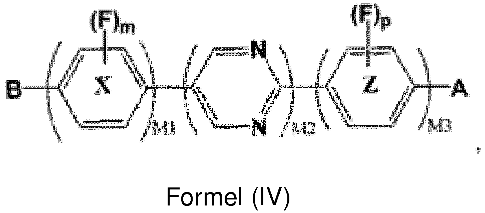 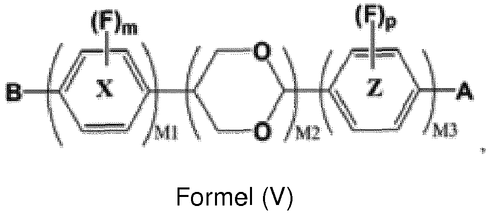 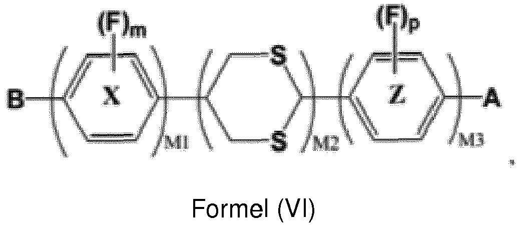 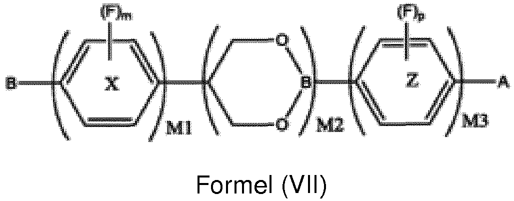
worin für die Formeln (III)-(VII) m und p unabhängig voneinander 0, 1, 2, 3 oder 4 sind, M2 1 oder 2 ist und M3 1 oder 2 ist;
X und Z substituierte oder unsubstituierte Phenylringe sind,
F ein Fluoratom ist, das eines oder mehrere der Wasserstoffatome am Phenylring von X oder Z substituiert; und
für die Formeln (III)-(IV) und (VI)-(VII) M1 0, 1 oder 2 ist; und
für Formel (V) M1 1 oder 2 ist; und
für Formel (III) B ausgewählt ist aus der Gruppe, bestehend aus: CN, F, NCS, NCO, CF₃, CHF₂, CH₂F, OCF₃, OCHF₂, OCH₂F, NO₂, Cl, CH=CF₂ und OCH=CF₂; C1-C20 Alkyl, C2-C20 Alkenyloxy, C1-C20 Silanyl und C1-C20 Siloxanyl sowie deren halogenierten Gruppen; und A ausgewählt ist aus der Gruppe, bestehend aus CN, F, NCS, NCO, CF₃, CHF₂, CH₂F, OCF₃, OCHF₂, OCH₂F, NO₂, Cl, CH=CF₂ und OCH=CF₂; C1-C20 Alkyl, C2-C20 Alkenyl, C2-C20 Alkenyloxy, C1-C20 Silanyl und C1-C20 Siloxanyl sowie deren halogenierten Gruppen;
für Formel (IV) B ausgewählt ist aus der Gruppe, bestehend aus: CN, F, NCS, NCO, CF₃, CHF₂, CH₂F, OCF₃, OCHF₂, OCH₂F, NO₂, Cl, CH=CF₂ und OCH=CF₂; C2-C20 Alkenyloxy, C1-C20 Silanyl und C1-C20 Siloxanyl und deren halogenierten Gruppen; und A ausgewählt ist aus der Gruppe, bestehend aus: F, NCS, NCO, CF₃, CHF₂, CH₂F, OCF₃, OCHF₂, OCH₂F, NO₂, Cl, CH=CF₂ und OCH=CF₂; C2-C20 Alkenyl, C2-C20 Alkenyloxy, C1-C20 Silanyl und C1-C20 Siloxanyl und deren halogenierten Gruppen; und
für Formel (V) B ausgewählt ist aus der Gruppe, bestehend aus: CN, F, NCS, NCO, CF₃, CHF₂, CH₂F, OCF₃, OCHF₂, OCH₂F, NO₂, Cl, CH=CF₂ und OCH=CF₂; C1-C20 Alkyl, C1-C20 Alkoxy, C2-C20 Alkenyloxy, C1-C20 Silanyl und C1-C20 Siloxanyl sowie deren halogenierten Gruppen; und A ausgewählt ist aus der Gruppe, bestehend aus: CN, F, NCO, CF₃, CHF₂, CH₂F, OCF₃, OCHF₂, OCH₂F, NO₂, Cl, CH=CF₂ und OCH=CF₂; C1-C20 Alkyl, C2-C20 Alkenyl, C2-C20 Alkenyloxy, C1-C20 Silanyl und C1-C20 Siloxanyl sowie deren halogenierten Gruppen; und
für Formel (VI) B ausgewählt ist aus der Gruppe, bestehend aus: CN, F, NCS, NCO, CF₃, CHF₂, CH₂F, OCF₃, OCHF₂, OCH₂F, NO₂, Cl, CH=CF₂ und OCH=CF₂; C1-C20 Alkyl, C2-C20 Alkenyloxy, C1-C20 Silanyl und C1-C20 Siloxanyl sowie deren halogenierten Gruppen; und A ausgewählt ist aus der Gruppe, bestehend aus: F, NCS, NCO, CF₃, CHF₂, CH₂F, OCF₃, OCHF₂, OCH₂F, NO₂, Cl, CH=CF₂ und OCH=CF₂; C1-C20 Alkyl, C2-C20 Alkenyl, C2-C20 Alkenyloxy, C1-C20 Silanyl und C1-C20 Siloxanyl sowie deren halogenierten Gruppen; und
für Formel (VII) B ausgewählt ist aus der Gruppe, bestehend aus: CN, F, NCS, NCO, CF₃, CHF₂, CH₂F, OCF₃, OCHF₂, OCH₂F, NO₂, Cl, CH=CF₂ und OCH=CF₂; C1-C20 Alkyl, C1-C20 Alkoxy, C2-C20 Alkenyl, C2-C20 Alkenyloxy, C1-C20 Silanyl und C1-C20 Siloxanyl sowie deren halogenierten Gruppen; und A ausgewählt ist aus der Gruppe, bestehend aus: F, NCS, NCO, CF₃, CHF₂, CH₂F, OCF₃, OCHF₂, OCH₂F, NO₂, Cl und OCH=CF₂; C2-C20 Alkenyl, C2-C20 Alkenyloxy, C1-C20 Silanyl und C1-C20 Siloxanyl;
und wobei die Vorrichtung einen Kondensator aufweist, der in der Lage ist, elektrische Energie in zwei Wellenformen anzulegen, wobei eine Wellenform Hochfrequenz-Treiberimpulse für den Löschvorgang und die andere Wellenform Niedrigfrequenz-Treiberimpulse für den Frostbetrieb sind, und
wobei das smektische A-Phasen-Flüssigkristallmaterial ferner mindestens eine Esterverbindung der Formel (II) umfasst: 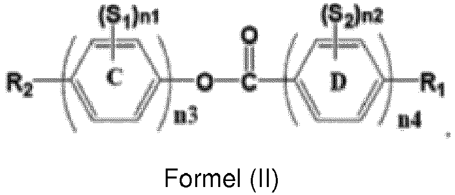 worin
n₁ und n₂ unabhängig voneinander 0, 1, 2, 3 oder 4 sind,
n₃ 0, 1 oder 2 ist und n₄ 0, 1, 2 oder 3 ist;
C und D unabhängig voneinander substituierte oder unsubstituierte Phenylringe sind;
S₁ und S₂ unabhängig voneinander ein N-Atom oder ein F-Atom sind, wobei eines oder mehrere der C-Atome am Phenylring ersetzt werden, wenn S₁ oder S₂ ein N-Atom ist, und eines oder mehrere der H-Atome am Phenylring ersetzt werden, wenn S₁ oder S₂ ein F-Atom ist;
R₁ und R₂ unabhängig voneinander ausgewählt sind aus der Gruppe, bestehend aus: F, NCS, NCO, CF₃, CHF₂, CH₂F, OCF₃, OCHF₂, OCH₂F, NO₂, Cl, CH=CF₂ und OCH=CF₂; C1-C20 Alkyl, C1-C20 Alkoxy, C1-C20 Alkenyl, C1-C20 Alkenyloxy, C1-C20 Silanyl und C1-C20 Siloxanyl sowie deren halogenierten Gruppen; C1-C20 Alkyl, C1-C20 Alkoxy, C1-C20-Alkenyl, C1-C20 Alkenyloxy und Isomeren davon, wobei ein beliebiges -CH₂-, substituiert ist mit -O-, -S-, -CF₂, -CF₂O-, - CO-, -O-COO-, -CF=CF-, -CH=CF-, -CF=CH- und -CH=CH-.

9. Vorrichtung nach Anspruch 8, wobei die Esterverbindung der Formel (II) mindestens eine der Verbindungen der Formeln (VIII), (XIII) und (XIV) ist, 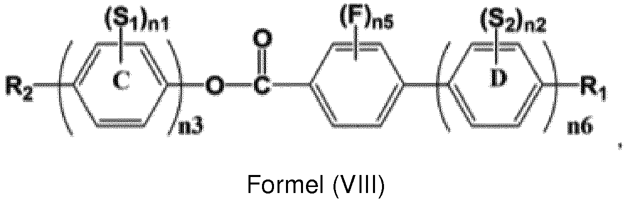 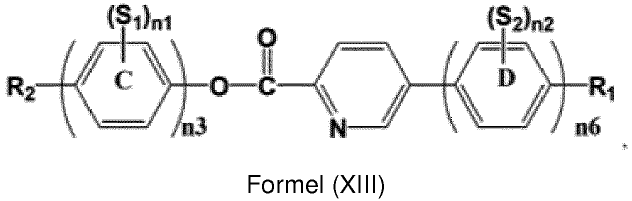 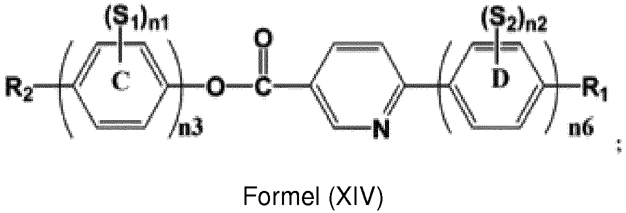 worin n₅ 0, 1, 2, 3, oder 4 ist und n₆ 0, 1 oder 2 ist.

## Revendications

1. Matériau à cristaux liquides en phase smectique A, comprenant au moins un composé hétérocyclique de formule (III), (IV), (V), (VI) ou (VII) : 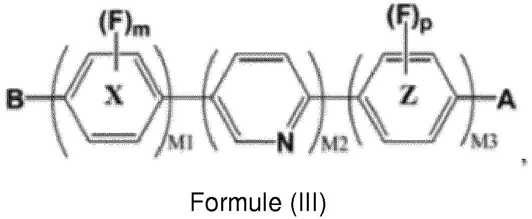 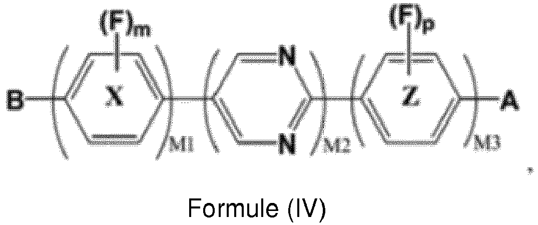 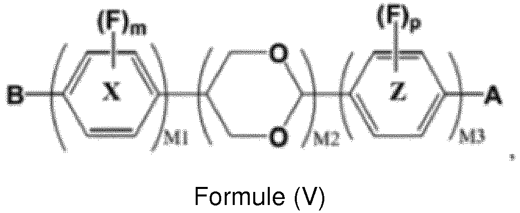 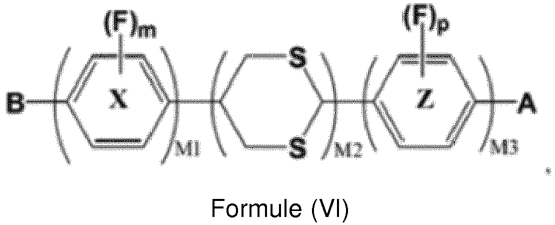 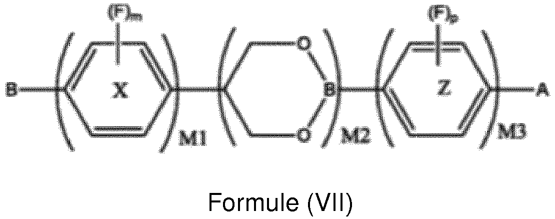
dans lesquelles pour les formules (III) à (VII), m et p valent indépendamment 0, 1, 2, 3 ou 4, M2 vaut 1 ou 2 et M3 vaut 1 ou 2 ;
X et Z sont des cycles phényle substitués ou non substitués,
F est un atome de fluor, substituant l'un quelconque ou plusieurs des atomes d'hydrogène sur le cycle phényle de X ou Z ; et
pour les formules (III) à (IV) et (VI) à (VII), M1 vaut 0, 1 ou 2 ; et
pour la formule (V), M1 vaut 1 ou 2 ; et
pour la formule (III), B est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcényloxy en C2 à C20, silanyle en C1 à C20 et siloxanyle en C1 à C20, et leurs groupes halogénés ; et A est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcényle en C2 à C20, alcényloxy en C2 à C20, silanyle en C1 à C20 et siloxanyle en C1 à C20, et leurs groupes halogénés ;
pour la formule (IV), B est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alcényloxy en C2 à C20, silanyle en C1 à C20 et siloxanyle en C1 à C20, et leurs groupes halogénés ; et A est choisi dans le groupe constitué par : les groupes F, NCS, NCO, CF3, CHF₂, CH₂F, OCF₃, OCHF₂, OCH₂F, NO₂, Cl, CH=CF₂ et OCH=CF₂ ; alcényle en C2 à C20, alcényloxy en C2 à C20, silanyle en C1 à C20 et siloxanyle en C1 à C20 et leurs groupes halogénés ; et
pour la formule (V), B est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcoxy en C1 à C20, alcényloxy en C2 à C20, silanyle en C1 à C20 et siloxanyle en C1 à C20 et leurs groupes halogénés ; et A est choisi dans le groupe constitué par : les groupes CN, F, NCO, CF₃, CHF₂, CH₂F, OCF₃, OCHF₂, OCH₂F, NO₂, Cl, CH=CF₂ et OCH=CF₂ ; alkyle en C1 à C20, alcényle en C2 à C20, alcényloxy en C2 à C20, silanyle en C1 à C20, siloxanyle en C1 à C20 et leurs groupes halogénés ; et
pour la formule (VI), B est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcényloxy en C2 à C20, silanyle en C1 à C20 et siloxanyle en C1 à C20 et leurs groupes halogénés ; et A est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcényle en C2 à C20, alcényloxy en C2 à C20, silanyle en C1 à C20 et siloxanyle en C1 à C20 et leurs groupes halogénés ; et
pour la formule (VII), B est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcoxy en C1 à C20, alcényle en C2 à C20, alcényloxy en C2 à C20, silanyle en C1 à C20 et siloxanyle en C1 à C20 et leurs groupes halogénés ; et A est choisi dans le groupe constitué par : les groupes F, NCS, NCO, CF₃, CHF₂, CH₂F, OCF₃, OCHF₂, OCH₂F, NO₂, Cl, CH=CF₂ et OCH=CF₂ ; alcényle en C2 à C20, alcényloxy en C2 à C20, silanyle en C1 à C20 et siloxanyle en C1 à C20 ;
et où le matériau à cristaux liquides en phase smectique A comprend en outre au moins un composé ester de formule (II) : 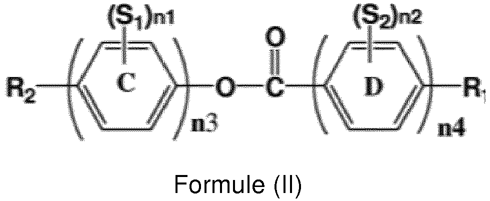
dans laquelle n₁ et n₂ valent indépendamment 0, 1, 2, 3 ou 4,
n₃ vaut 0, 1 ou 2 et n₄ vaut 0, 1, 2 ou 3 ;
C et D sont indépendamment des cycles phényle substitués ou non substitués ;
S₁ et S₂ sont indépendamment un atome N ou un atome F, remplaçant l'un quelconque ou plusieurs des atomes C sur le cycle phényle quand S₁ ou S₂ est un atome N, et remplaçant l'un quelconque ou plusieurs des atomes H sur le cycle phényle quand S₁ ou S₂ est un atome F ;
R₁ et R₂ sont choisis indépendamment dans le groupe constitué par : les groupes F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcoxy en C1 à C20, alcényle en C1 à C20, alcényloxy en C1 à C20, silanyle en C1 à C20 et siloxanyle en C1 à C20 et leurs groupes halogénés ; alkyle en C1 à C20, alcoxy en C1 à C20, alcényle en C1 à C20, alcényloxy en C1 à C20 et leurs isomères avec un quelconque -CH₂- substitué avec - O-, -S-, un groupe -CF₂-, -CF₂O-, -CO-, -O-COO-, -CF=F, -CH=CF-, - CF=CH- et CH=CH-.

2. Matériau à cristaux liquides en phase smectique A selon la revendication 1, comprenant en outre un composé ionique.

3. Matériau à cristaux liquides en phase smectique A selon la revendication 2, comprenant en outre une formation de cristal liquide nématique, des monomères de cristal liquide nématique ou des composés de type tige.

4. Matériau à cristaux liquides en phase smectique A selon la revendication 3, comprenant en outre un colorant dichroïque ou une colle à UV.

5. Matériau à cristaux liquides en phase smectique A selon la revendication 1, dans lequel le composé ester de formule (II) est au moins un des composés de formules (VIII, (XIII) et (XIV), 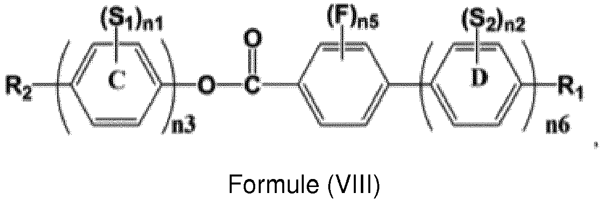 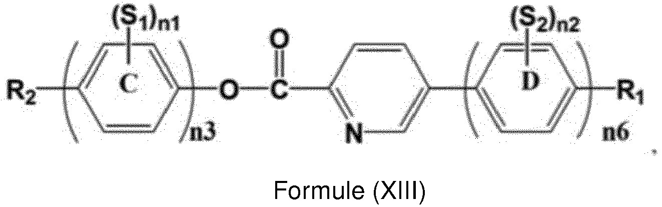 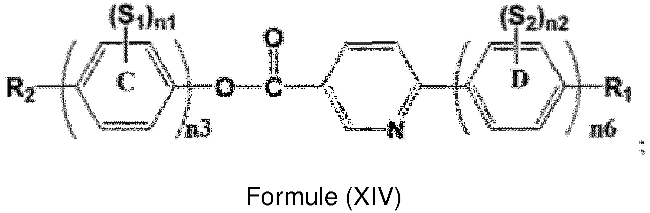 dans lesquelles n₅ vaut 0, 1, 2, 3 ou 4 et n₆ vaut 0, 1 ou 2.

6. Matériau à cristaux liquides en phase smectique A selon la revendication 2, dans lequel le composé ionique est choisi dans le groupe constitué par : le lauryl sulfate de sodium, l'iodure d'éthyltriphénylphosphonium ; l'iodure de (ferrocénylméthyl)triméthylammonium, l'hexafluorophosphate de 1,2-diméthyl-3-butylimidazole, le paratoluènesulfonate de tétraéthylamine, l'iodure de phényltriéthylammonium, l'hexafluorophosphate de 1-octyl-3-méthylimidazole, le bis(tétra-n-butylamine)bis(1,3-dithiole-2-thione-4,5-dithiol)palladium, le bis(1,3-dithiole-2-thione-4,5-dithiol)nickel de tétra-n-butyle, le bis(1,3-dithiole-2-thiol-4,5dithiol)zinc de bis(tétra-n-butylammonium), le bis(tétra-n-butylamonium)tétracyanodiphénoquinodiméthane, le bromure de tétrabutylammonium, le perchlorate de cétylammonium, le bromure de cétyltétraammonium, le ferrate tétrachlorure de 1-butyl-3-méthylimidazole, l'iodure de méthyltriphénylphosphonium, l'iodure de tétraphénylphosphonium, le bromure de cétyltriméthyl ammonium, le perchlorate de cétyltriméthyl ammonium, le chlorure de cétyldiméthylbenzylammonium, le bromure de dodécyl pyridine, le bromure de cétyl pyridine, le chlorure de cétyl pyridine et le bromure de cétyltributylammonium.

7. Matériau à cristaux liquides en phase smectique A selon la revendication 6, comprenant en outre un composé ionique choisi dans le groupe constitué par : le lauryl sulfate de sodium, l'iodure d'éthyltriphénylphosphonium ; l'iodure de (ferrocénylméthyl)triméthylammonium, l'hexafluorophosphate de 1,2-diméthyl-3-butylimidazole, le paratoluènesulfonate de tétraéthylamine, l'iodure de phényltriéthylammonium, l'hexafluorophosphate de 1-octyl-3-méthylimidazole, le bis(tétra-n-butylamine)bis(1,3-dithiole-2-thione-4,5-dithiol)palladium, le bis(1,3-dithiole-2-thione-4,5-dithiol)nickel de tétra-n-butyle, le bis(1,3-dithiole-2-thiol-4,5-dithiol)zinc de bis(tétra-n-butylammonium), le bis(tétra-n-butylamonium)tétracyanodiphénoquinodiméthane, le bromure de tétrabutylammonium, le perchlorate de cétylammonium, le bromure de cétyltétraammonium, le ferrate tétrachlorure de 1-butyl-3-méthylimidazole, l'iodure de méthyltriphénylphosphonium, l'iodure de tétraphénylphosphonium, le bromure de cétyltriméthyl ammonium, le perchlorate de cétyltriméthyl ammonium, le chlorure de cétyldiméthylbenzylammonium, le bromure de dodécyl pyridine, le bromure de cétyl pyridine, le chlorure de cétyl pyridine et le bromure de cétyltributylammonium.

8. Dispositif comprenant un matériau à cristaux liquides en phase smectique A,
dans lequel le matériau à cristaux liquides en phase smectique A comprend au moins un composé hétérocyclique de formule (III), (IV), (V), (VI) ou (VII) : 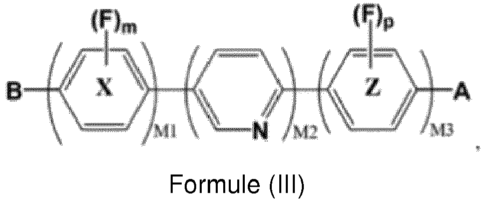 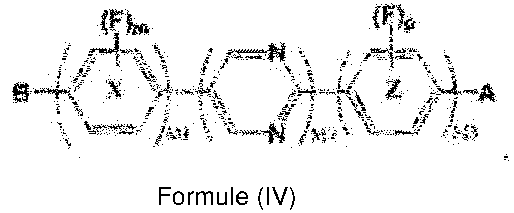 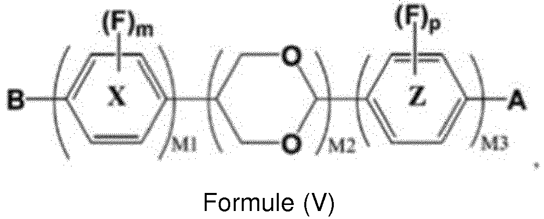 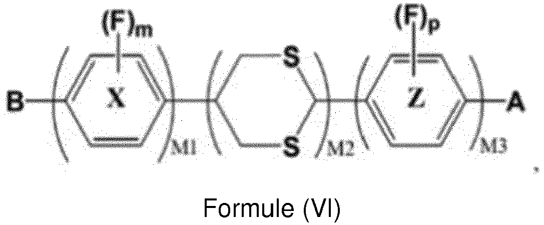 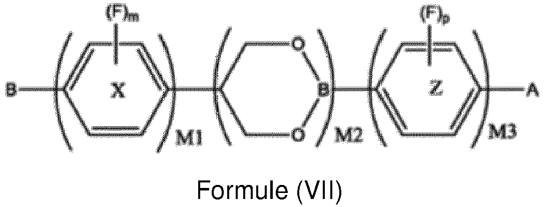
dans lesquelles pour les formules (III) à (VII), m et p valent indépendamment 0, 1, 2, 3 ou 4, M2 vaut 1 ou 2 et M3 vaut 1 ou 2 ;
X et Z sont des cycles phényle substitués ou non substitués,
F est un atome de fluor, substituant l'un quelconque ou plusieurs des atomes d'hydrogène sur le cycle phényle de X ou Z ; et
pour les formules (III) à (IV) et (VI) à (VII), M1 vaut 0, 1 ou 2 ; et
pour la formule (V), M1 vaut 1 ou 2 ; et
pour la formule (III), B est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcényloxy en C2 à C20, silanyle en C1 à C20 et siloxanyle en C1 à C20, et leurs groupes halogénés ; et A est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcényle en C2 à C20, alcényloxy en C2 à C20, silanyle en C1 à C20 et siloxanyle en C1 à C20, et leurs groupes halogénés ;
pour la formule (IV), B est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alcényloxy en C2 à C20, silanyle en C1 à C20 et siloxanyle en C1 à C20, et leurs groupes halogénés ; et A est choisi dans le groupe constitué par : les groupes F, NCS, NCO, CF₃, CHF₂, CH₂F, OCF₃, OCHF₂, OCH₂F, NO₂, Cl, CH=CF₂ et OCH=CF₂ ; alcényle en C2 à C20, alcényloxy en C2 à C20, silanyle en C1 à C20 et siloxanyle en C1 à C20 et leurs groupes halogénés ; et
pour la formule (V), B est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcoxy en C1 à C20, alcényloxy en C2 à C20, silanyle en C1 à C20 et siloxanyle en C1 à C20 et leurs groupes halogénés ; et A est choisi dans le groupe constitué par : les groupes CN, F, NCO, CF₃, CHF₂, CH₂F, OCF₃, OCHF₂, OCH₂F, NO₂, Cl, CH=CF₂ et OCH=CF₂ ; alkyle en C1 à C20, alcényle en C2 à C20, alcényloxy en C2 à C20, silanyle en C1 à C20 et siloxanyle en C1 à C20 et leurs groupes halogénés ; et
pour la formule (VI), B est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcényloxy en C2 à C20, silanyle en C1 à C20 et siloxanyle en C1 à C20 et leurs groupes halogénés ; et A est choisi dans le groupe constitué par : les groupes F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcényloxy en C2 à C20, silanyle en C1 à C20 et siloxanyle en C1 à C20 et leurs groupes halogénés ; et
pour la formule (VII), B est choisi dans le groupe constitué par : les groupes CN, F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcoxy en C1 à C20, alcényle en C2 à C20, alcényloxy en C2 à C20, silanyle en C1 à C20 et siloxanyle en C1 à C20 et leurs groupes halogénés ; et A est choisi dans le groupe constitué par : les groupes F, NCS, NCO, CF₃, CHF₂, CH₂F, OCF₃, OCHF₂, OCH₂F, NO₂, Cl, CH=CF₂ et OCH=CF₂ ; alcényle en C2 à C20, alcényloxy en C2 à C20, silanyle en C1 à C20 et siloxanyle en C1 à C20,
et où le dispositif a un condensateur capable d'appliquer de l'énergie électrique en formes d'onde doubles, une forme d'onde est des impulsions de direction à haute fréquence pour l'opération d'éclaircissement, et l'autre forme d'onde est des impulsions de direction à basse fréquence pour l'opération de dépolissage, et
où le matériau à cristaux liquides en phase smectique A comprend en outre un composé ester de formule (II) : 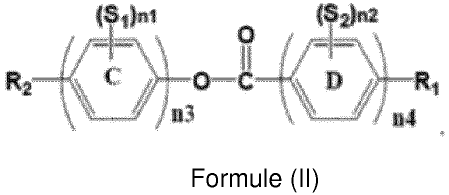 dans laquelle
n₁ et n₂ valent indépendamment 0, 1, 2, 3 ou 4,
n₃ vaut 0, 1 ou 2 et n₄ vaut 0, 1, 2 ou 3 ;
C et D sont indépendamment des cycles phényle substitués ou non substitués ;
S₁ et S₂ sont indépendamment un atome N ou un atome F, remplaçant l'un quelconque ou plusieurs des atomes C sur le cycle phényle quand S₁ ou S₂ est un atome N, et remplaçant l'un quelconque ou plusieurs des atomes H sur le cycle phényle quand S₁ ou S₂ est un atome F ; et
R₁ et R₂ sont choisis indépendamment dans le groupe constitué par : les groupes F, NCS, NCO, CF₃, CHF₂, CH₂F, OCF₃, OCHF₂, OCH₂F, NO₂, Cl, CH=CF₂ et OCH=CF₂ ; alkyle en C1 à C20, alcoxy en C1 à C20, alcényle en C1 à C20, alcényloxy en C1 à C20, silanyle en C1 à C20 et siloxanyle en C1 à C20 et leurs groupes halogénés ; alkyle en C1 à C20, alcoxy en C1 à C20, alcényle en C1 à C20, alcényloxy en C1 à C20 et leurs isomères avec un quelconque -CH₂- substitué avec - O-, -S-, un groupe -CF₂-, -CF₂O-, -CO-, -O-COO-, -CF=F, -CH=CF-, - CF=CH- et CH=CH-.

9. Dispositif selon la revendication 8, dans lequel le composé ester de formule (II) est au moins un des composés de formule (VIII), (XIII) et (XIV), 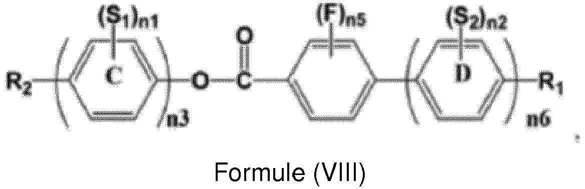 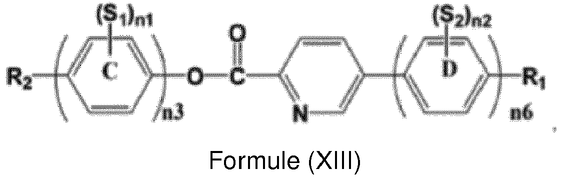 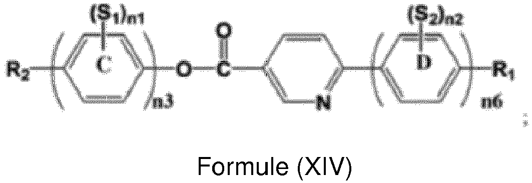 dans lesquelles n₅ vaut 0, 1, 2, 3 ou 4 et n₆ vaut 0, 1 ou 2.
